# EUROPEAN PATENT SPECIFICATION

(11) **EP 4 211 466 B1**
(45) Date of publication and mention of the grant of the patent: **26.02.2025**
(21) Application number: 21786050.1
(22) Date of filing: 27.08.2021
(51) Int. Cl.: G01N 33/53, C12Q 1/6869

(54) **DETECTING NUCLEIC ACIDS USING SUGAR-LECTIN COUPLINGS**
NACHWEIS VON NUKLEINSÄUREN MITTELS ZUCKER-LEKTIN-KOPPLUNGEN
DÉTECTION D'ACIDES NUCLÉIQUES À L'AIDE DE COUPLAGES SUCRE-LECTINE

(30) Priority: 11.09.2020 US 202063077416 P
(43) Date of publication of application: 19.07.2023
(73) Proprietor: Illumina, Inc., San Diego, CA 92122 (US); Illumina Singapore Pte Ltd, Singapore 757716 (SG)
(72) Inventor: PANTOJA, Rigo, San Diego, California 92122 (US); ORTIZ, Daniel, San Diego, California 92122 (US); YANG, Xiangyuan, 757716 Singapore (SG); TEO, Yin Nah, 757716 Singapore (SG); VERMAAS, Eric, San Diego, California 92122 (US); ECKHARDT, Allen, San Diego, California 92122 (US)
(74) Representative: Murgitroyd & Company
(86) International application number: PCT/US2021/047978
(87) International publication number: WO 2022/055726

(56) References cited:
- YU JU-CHING ET AL: "An integrated microfluidic system using mannose-binding lectin for bacteria isolation and biofilm-related gene detection", MICROFLUIDICS AND NANOFLUIDICS, SPRINGER BERLIN HEIDELBERG, BERLIN/HEIDELBERG, vol. 22, no. 1, 9 January 2018 (2018-01-09), pages 1 - 10, XP036401829, ISSN: 1613-4982, [retrieved on 20180109], DOI: 10.1007/S10404-017-2031-3
- ZHANG JING ET AL: "Specific recognition of lectins by oligonucleotide glycoconjugates and sorting on a DNA microarray", CHEMICAL COMMUNICATIONS, no. 44, 1 January 2009 (2009-01-01), UK, pages 6795, XP055856576, ISSN: 1359-7345, Retrieved from the Internet <URL:https://pubs.rsc.org/en/content/articlepdf/2009/cc/b915132j> DOI: 10.1039/b915132j
- CHRISTIAN SCHEIBE ET AL: "DNA-programmed spatial screening of carbohydrate–lectin interactions", CHEMICAL SCIENCE, vol. 2, no. 4, 1 January 2011 (2011-01-01), United Kingdom, pages 770, XP055272631, ISSN: 2041-6520, DOI: 10.1039/c0sc00565g
- SCHEIBE CHRISTIAN ET AL: "Supporting Information: DNA-programmed spatial screening of carbohydrate-lectin interactions", 1 January 2011 (2011-01-01), pages 1 - 32, XP055857903, Retrieved from the Internet <URL:https://www.rsc.org/suppdata/sc/c0/c0sc00565g/c0sc00565g.pdf?_ga=2.2456688.1936456175.1636023036-389611948.1631526273> [retrieved on 20211104]
- ZHANG X Q ET AL: "Galactosylated ternary DNA/polyphosphoramidate nanoparticles mediate high gene transfection efficiency in hepatocytes", JOURNAL OF CONTROLLED RELEASE, ELSEVIER, AMSTERDAM, NL, vol. 102, no. 3, 16 February 2005 (2005-02-16), pages 749 - 763, XP027664458, ISSN: 0168-3659, [retrieved on 20050216]
- FENG YIMEI ET AL: "Lectin-mediated in situ rolling circle amplification on exosomes for probing cancer-related glycan pattern", ANALYTICA CHIMICA ACTA, ELSEVIER, AMSTERDAM, NL, vol. 1039, 19 July 2018 (2018-07-19), pages 108 - 115, XP085505074, ISSN: 0003-2670, DOI: 10.1016/J.ACA.2018.07.040

## Description

### BACKGROUND

The detection of specific nucleic acid sequences present in a biological sample has been used, for example, as a method for identifying and classifying microorganisms, diagnosing infectious diseases, detecting and characterizing genetic abnormalities, identifying genetic changes associated with cancer, studying genetic susceptibility to diseases, and measuring response to various types of treatment. A common technique for detecting specific nucleic acid sequences in a biological sample is nucleic acid sequencing.

Nucleic acid sequencing methodology has evolved from the chemical degradation methods used by Maxam and Gilbert and the strand elongation methods used by Sanger. Several sequencing methodologies are now in use which allow for the parallel processing of thousands of nucleic acids all on a single chip. Some platforms include bead-based and microarray formats in which silica beads are functionalized with probes depending on the application of such formats in applications including sequencing, genotyping, or gene expression profiling.

Some sequencing systems use fluorescence-based detection, whether for "sequencing-by-synthesis" or for genotyping, in which a given nucleotide is labeled with a fluorescent label, and the nucleotide is identified based on detecting the fluorescence from that label.

Yu Ju-Ching et a, Microfluidics and Nanofluidics, 22(1) (2018) 1-10 describes an integrated microfluidic system using mannose-binding lectin for bacteria isolation and biofilm-related gene detection. Zhang J et al, Chemical Communications 44 (2009) page 6795 describes the specific recognition of lectins by oligonucleotide glycoconjugates and sorting at the surface of micro-reactors bearing the immobilised complementary DNA sequences. Scheibe et al, Chemical Science 2(4) (2011) 770-775 describes DNA-programmed spatial screening of carbohydrate-lectin interactions. Zhang X Q et al, Journal of Controlled Release 102(3) (2005) 749-763 describes the synthesis of galactosylated polyphosphoramidates (Gal-PPAs) with different ligand substitution degrees and their evaluation as hepatocyte-targeted gene carriers. Feng et al, Analytica Chimica Acta 1039 (2018) 108-115 describes the fabrication of an exosomal array for probing cancer-related exosomal glycan signatures by lectin recognition-mediated in situ rolling circle assembly of fluorophore-labeled DNA.

### SUMMARY

Provided in some examples herein is a method for detecting an element. The method includes coupling a first element-sugar complex to a first substrate. The first element-sugar complex includes a first element coupled to a first sugar. The method includes coupling a first lectin to the first sugar; and detecting the first element using at least detection of the first lectin.

The first element includes an analyte. The analyte includes a first nucleotide. Coupling the first element-sugar complex to the first substrate includes incorporating the first nucleotide into a first oligonucleotide coupled to the first substrate. The first oligonucleotide is hybridized to a second oligonucleotide, and incorporating the first nucleotide into the first oligonucleotide includes extending the first oligonucleotide using at least a sequence of the second oligonucleotide. In some examples, coupling the first element-sugar complex to the first substrate includes flowing a solution over the substrate. The solution may include the first element-sugar complex; and a second element-sugar complex including a second element coupled to a second sugar. The first lectin selectively binds the first sugar and does not bind the second sugar. The first and second elements may include different nucleotide types than one another. In some examples, the first and second sugars include different sugar types than one another.

In some examples, the method further includes coupling the second element-sugar complex to the first substrate or to a second substrate; coupling a second lectin to the second sugar; and detecting the second element using at least detection of the second lectin. In some examples, the solution further includes a third element-sugar complex including a third element coupled to a third sugar; and a fourth element-sugar complex including a fourth element coupled to a fourth sugar. The method further may include coupling the third element-sugar complex to the first substrate, to the second substrate, or to a third substrate. The method further may include coupling a third lectin to the third sugar; and detecting the third element using at least detection of the third lectin. The method further may include coupling the fourth element-sugar complex to the first substrate, to the second substrate, to the third substrate, or to a fourth substrate. The method further may include coupling a fourth lectin to the fourth sugar; and detecting the fourth element using at least detection of the fourth lectin.

In some examples, the first element sugar-complex further includes a fifth sugar coupled to the element. In some examples, the method further includes coupling the first lectin to the fifth sugar.

In some examples, the method further includes coupling a first fluorophore to the first lectin, and detection of the first lectin includes detecting fluorescence from the first fluorophore. In some examples, the first fluorophore includes a first plurality of fluorophores. In some examples, coupling the first fluorophore to the first lectin includes coupling a polymer to the first lectin, the polymer includes the first fluorophore and a sixth sugar, and the first lectin couples to the sixth sugar. In some examples, the polymer includes a bead. In some examples, the polymer includes a polypeptide. In some examples, the first fluorophore is coupled to the first lectin before coupling the first lectin to the first sugar. In some examples, the first fluorophore is coupled to the first lectin after coupling the first lectin to the first sugar.

In some examples, a seventh sugar is coupled to the first lectin, a second lectin is coupled to the seventh sugar, and detection of the first lectin includes detection of the second lectin. In some examples, a second fluorophore is coupled to the second lectin, and detection of the first lectin includes detecting fluorescence from the second fluorophore. In some examples, the first fluorophore is a different type of fluorophore than the second fluorophore. In some examples, the first lectin includes Concanavalin A (Con A), the seventh sugar includes N-acetyl-galactosamine (GalNAc), and the second lectin includes soybean agglutinin (SBA).

In some examples, the first substrate includes a bead.

In some examples, the first sugar includes an alkyl sugar.

In some examples, the first sugar includes a monosaccharide.

In some examples, the first sugar includes a disaccharide.

In some examples, the first lectin includes Concanavalin A (Con A) and the first sugar includes mannose (Man) or glucose (Glc); the first lectin includes wheat germ agglutinin (WGA) and the first sugar includes N-acetyl-glucosamine (GlcNAc) or N-acetyl-neuraminic acid (Neu5Ac); the first lectin includes soybean agglutinin (SBA) and the first sugar includes galactose (Gal) or N-acetyl-galactosamine (GalNAc); the first lectin includes *Dolichos bifloris* (DBA) and the first sugar includes GalNAcα3GalNAc or GalNAc; the first lectin includes Ricin and the first sugar includes galactose; the first lectin includes peanut agglutinin (PNA) and the first sugar includes galactose or Galβ3GalNAcα (T-antigen); the first lectin includes *Pisum sativum* (PSA) and the first sugar includes mannose or glucose; the first lectin includes *Lens culinaris* (LCA) and the first sugar includes mannose or glucose; the first lectin includes *Galanthus nivalus* (GNA) and the first sugar includes mannose; the first lectin includes *Solanum tuberosum* (STA) and the first sugar includes (GlcNAc)ₙ; the first lectin includes Asialoglycoprotein receptor (ASGPR) H1 and the first sugar includes galactose; the first lectin includes Galectin-3 and the first sugar includes galactose; the first lectin includes Sialoadhesin and the first sugar includes Neu5Ac; the first lectin includes Cation-dependent mannose-6-phosphate receptor (CD-MPR) and the first sugar includes Man6P; or the first lectin includes C-reactive protein (CRP) and the first sugar includes galactose, Gal6P, or galacturonic acid.

Provided in some examples herein is a system as claimed in claim 12. The system includes a substrate, and a first element-sugar complex coupled to the substrate. The first element-sugar complex includes a first element coupled to a first sugar. The system includes a first lectin coupled to the first sugar. The system includes detection circuitry to detect the first element using at least detection of the first lectin.

The first element includes an analyteThe analyte includes a first nucleotide. The first element-sugar complex is coupled to the first substrate via incorporation of the first nucleotide into a first oligonucleotide coupled to the first substrate. The first oligonucleotide is hybridized to a second oligonucleotide, and the incorporation of the first nucleotide into the first oligonucleotide includes extending the first oligonucleotide using at least a sequence of the second oligonucleotide.

In some examples, the system further includes a solution to flow over the substrate. The solution may include the first element-sugar complex; and a second element-sugar complex including a second element coupled to a second sugar. The first lectin may selectively bind the first sugar and does not bind the second sugar. The first and second elements may include different nucleotide types than one another. In some examples, the first and second sugars include different sugar types than one another. In some examples, the second element-sugar complex is coupled to the first substrate or to a second substrate; a second lectin is coupled to the second sugar; and the detection circuitry is to detect the second element using at least detection of the second lectin.

In some examples, the solution further includes a third element-sugar complex including a third element coupled to a third sugar, and a fourth element-sugar complex including a fourth element coupled to a fourth sugar. The third element-sugar complex may be coupled to the first substrate, to the second substrate, or to a third substrate. A third lectin may be coupled to the third sugar. The detection circuitry may be to detect the third element using at least detection of the third lectin. The fourth element-sugar complex may be coupled to the first substrate, to the second substrate, to the third substrate, or to a fourth substrate. A fourth lectin may be coupled to the fourth sugar. The detection circuitry may be to detect the fourth element using at least detection of the fourth lectin.

In some examples, the first element sugar-complex further includes a fifth sugar coupled to the element. In some examples, the first lectin is coupled to the fifth sugar.

In some examples, a first fluorophore is coupled to the first lectin, and the detection circuitry is to detect the first lectin using at least detection of fluorescence from the first fluorophore. In some examples, the first fluorophore includes a first plurality of fluorophores. In some examples, the first fluorophore is coupled to the first lectin using a polymer, the polymer includes the first fluorophore and a sixth sugar, and the first lectin couples to the sixth sugar. In some examples, the polymer includes a bead. In some examples, the polymer includes a polypeptide. In some examples, the first fluorophore is coupled to the first lectin before the first lectin is coupled to the first sugar. In some examples, the first fluorophore is coupled to the first lectin after the first lectin is coupled to the first sugar.

In some examples, a seventh sugar is coupled to the first lectin, a second lectin is coupled to the seventh sugar, and the detection circuitry is to detect the first lectin using at least detection of the second lectin.

In some examples, a second fluorophore is coupled to the second lectin, and the detection circuitry is to detect the first lectin using at least detecting fluorescence from the second fluorophore. In some examples, the first fluorophore is a different type of fluorophore than the second fluorophore.

In some examples, the first lectin includes Concanavalin A (Con A), the seventh sugar includes N-acetyl-galactosamine (GalNAc), and the second lectin includes soybean agglutinin (SBA). In some examples, the first lectin includes Concanavalin A (Con A) and the first sugar includes mannose (Man) or glucose (Glc); the first lectin includes wheat germ agglutinin (WGA) and the first sugar includes N-acetyl-glucosamine (GlcNAc) or N-acetyl-neuraminic acid (Neu5Ac); the first lectin includes soybean agglutinin (SBA) and the first sugar includes galactose (Gal) or N-acetyl-galactosamine (GalNAc); the first lectin includes *Dolichos bifloris* (DBA) and the first sugar includes GalNAcα3GalNAc or GalNAc; the first lectin includes Ricin and the first sugar includes galactose; the first lectin includes peanut agglutinin (PNA) and the first sugar includes galactose or Galβ3GalNAcα (T-antigen); the first lectin includes *Pisum sativum* (PSA) and the first sugar includes mannose or glucose; the first lectin includes *Lens culinaris* (LCA) and the first sugar includes mannose or glucose; the first lectin includes *Galanthus nivalus* (GNA) and the first sugar includes mannose; the first lectin includes *Solanum tuberosum* (STA) and the first sugar includes (GlcNAc)ₙ; the first lectin includes Asialoglycoprotein receptor (ASGPR) H1 and the first sugar includes galactose; the first lectin includes Galectin-3 and the first sugar includes galactose; the first lectin includes Sialoadhesin and the first sugar includes Neu5Ac; the first lectin includes Cation-dependent mannose-6-phosphate receptor (CD-MPR) and the first sugar includes Man6P; or the first lectin includes C-reactive protein (CRP) and the first sugar includes galactose, Gal6P, or galacturonic acid.

In some examples, the first substrate includes a bead.

In some examples, the first sugar includes an alkyl sugar.

In some examples, the first sugar includes a monosaccharide.

In some examples, the first sugar includes a disaccharide.

It is to be understood that any respective features/examples of each of the aspects of the disclosure as described herein may be implemented together in any appropriate combination, and that any features/examples from any one or more of these aspects may be implemented together with any of the features of the other aspect(s) as described herein in any appropriate combination to achieve the benefits as described herein.

### BRIEF DESCRIPTION OF DRAWINGS

FIGS. 1A-1B schematically illustrate example process flows for detecting an element using a sugar-lectin coupling.
FIGS. 2A-2B schematically illustrate additional example process flows for detecting elements using sugar-lectin couplings.
FIG. 2C schematically illustrates an example element coupled to multiple sugars.
FIG. 3 schematically illustrates another example process flow for detecting an element using sugar-lectin couplings.
FIGS. 4A-4D schematically illustrate an example process flow for detecting elements using a combination of sugar-lectin couplings and one or more other types of couplings.
FIGS. 5A-5C schematically illustrate another example process flow for detecting elements using sugar-lectin couplings.
FIGS. 6A-6C schematically illustrate another example process flow for detecting elements using sugar-lectin couplings.
FIG. 7 schematically illustrates another example process flow for detecting an element using a sugar-lectin coupling.
FIG. 8 schematically illustrates an example process flow for coupling sugars and fluorophores to a polypeptide.
FIG. 9 schematically illustrates an example process flow for coupling sugars to a bead.
FIG. 10 is a liquid chromatography mass spectrometry (LCMS) spectrum of an example nucleotide-sugar complex.
FIG. 11 is an image of a gel showing the results of incorporation of nucleotide-sugar complexes, into growing polynucleotides by polymerases.

### DETAILED DESCRIPTION

Detection of elements using sugar-lectin couplings is provided herein.

For example, the present application provides systems according to claim 12 and methods according to claim 1 for detecting elements by coupling detectable moieties to those elements via sugar-lectin couplings. The element to be detected is coupled to a sugar, while the detectable moiety may be coupled to a lectin that is specific to that sugar. The lectin is coupled to the sugar, thus indirectly coupling the element to the detectable moiety. The detectable moiety may be detected via suitable detection circuitry, and using at least such detection the lectin - and thus the element - may be detected. In some examples, the detectable moiety may include a fluorophore that may be detected via suitable optical detection circuitry, and using at least fluorescence from the fluorophore the lectin - and thus the element - may be detected. In some examples, the lectin may be coupled to multiple fluorophores so as to provide for enhanced optical detectability. In still further examples, multiple lectins may be coupled together that respectively include multiple fluorophores, thus providing for still further enhancements in optical detectability. However, it will be appreciated that in the current systems and methods, a lectin may be detected in any suitable manner and is not limited to detection via fluorescence.

Some terms used herein will be briefly explained. Then, some example systems and example methods for detecting elements using sugar-lectin couplings will be described.

### Terms

Unless defined otherwise, all technical and scientific terms used herein have the same meaning as is commonly understood by one of ordinary skill in the art. The use of the term "including" as well as other forms, such as "include," "includes," and "included," is not limiting. The use of the term "having" as well as other forms, such as "have," "has," and "had," is not limiting. As used in this specification, whether in a transitional phrase or in the body of the claim, the terms "comprise(s)" and "comprising" are to be interpreted as having an open-ended meaning. That is, the above terms are to be interpreted synonymously with the phrases "having at least" or "including at least." For example, when used in the context of a process, the term "comprising" means that the process includes at least the recited steps, but may include additional steps. When used in the context of a compound, composition, or device, the term "comprising" means that the compound, composition, or device includes at least the recited features or components, but may also include additional features or components.

The terms "substantially", "approximately", and "about" used throughout this Specification are used to describe and account for small fluctuations, such as due to variations in processing. For example, they can refer to less than or equal to ±5%, such as less than or equal to ±2%, such as less than or equal to ±1%, such as less than or equal to ±0.5%, such as less than or equal to ±0.2%, such as less than or equal to ±0.1%, such as less than or equal to ±0.05%.

As used herein, "sugar" is intended to mean a water-soluble carbohydrate. Sugars may include monosaccharides, disaccharides, and polysaccharides. Sugars may be naturally occurring, or non-naturally occurring. Examples of naturally occurring monosaccharides include mannose, glucose, fructose, galactose, ribose, and deoxyribose. Examples of naturally occurring disaccharides include lactose, sucrose, and maltose. Other example sugars may include N-acetyl-glucosamine (GlcNAc)ₙ, N-acetyl-neuraminic acid (Neu5Ac), N-acetyl-galactosamine (GalNAc), Galβ3GalNAcα, GalNAcα3GalNAc, Man6P, and galacturonic acid. Non-naturally occurring sugars may include naturally occurring sugars that are modified so as to modify, add, or remove one or more additional moieties. For example, an "alkyne sugar" may include a sugar that is coupled (e.g., via a linker such as PEG) to an alkyne group via which the sugar may be coupled to another element, such as a nucleotide. In another example, a "sugar alcohol" may include a sugar with one or more hydrogen removed or modified to add one or more hydroxyl group, e.g. sorbitol, mannitol, xylitol, maltitol, lactitol, and erythritol. The term "nonsugar" is intended to mean excluding any sugar.

As used herein, "lectin" is intended to mean a protein that selectively binds a particular sugar or sugars, and as such does not bind any other sugars. "Monovalent" lectins may bind a single sugar at a given time, while "divalent" lectins may bind two sugars at once, and "multivalent" lectins may bind two or more sugars at once. Lectins may be naturally occurring, or non-naturally occurring. Naturally occurring lectins may include plant lectins and animal lectins. Example plant lectins include Concanavalin A (Con A), which may be derived from jack bean and which selectively binds α-mannose, α-glucose, branched α-mannosidic structures, high-mannose type ligand motifs, and hybrid type and biantennary complex type N-glycans; wheat germ agglutinin (WGA), which may be derived from wheat and which selectively binds (GlcNAc)₁₋₃, Neu5Ac (sialic acid), and GlcNAcβ1-4GlcNAcβ1-4GlcNAc ligand motifs; soybean agglutinin (SBA), which may be derived from soybean and which selectively binds galactose and GalNAc; *Dolichos bifloris* (DBA) which may be derived from horse gram and which selectively binds GalNAcα3GalNAc and GalNAc; Ricin communis Aglutinin or ricine (RCA), which may be derived from castor bean and which selectively binds galactose and Galβ1-4GlycNAcβ1-R ligand motifs; Peanut agglutinin (PNA), which may be derived from peanut and which selectively binds galactose, Galβ3GalNAcα (T-antigen), and Galβ1-3GalNAcα1-Ser/Thr (T-antigen) ligand motifs; *Pisum sativum* (PSA), which may be derived from pea and which selectively binds mannose and glucose; *Lens culinaris* (LCA), which may be derived from lentil and which selectively binds mannose and glucose; LCH (lentil lectin), which also may be derived from lentil and which selectively binds a fucosylated core region of bi-and triantennary complex type N-glycans; *Galanthus nivalus* (GNA), which may be derived from snowdrop and which selectively binds mannose and α 1-3 and α 1-6 linked high mannose structures; Jacalin (AIL), which may be derived from *Artocarpus integrifolia* and which selectively binds (Sia)Galβ1-3GalNAcα1-Ser/Thr (T-Antigen) ligand motifs; hairy vetch lectin (VVL), which may be derived from *Vicia villosa* and which selectively binds GalNAcα-Ser/Thr (Tn-Antigen) ligand motifs; elderberry lectin (SNA), which may be derived from *Sambucus nigra* and which selectively binds Neu5Acα2-6Gal(NAc)-R ligand motifs; Maackia amurensis lectin (MAL), which may be derived from *Maackia amurensis* and which selectively binds Neu5Ac/Gcα2-3Galβ1-4GlcNAcβ1-R; Ulex europaeus agglutinin (UEA), which may be derived from *Ulex europaeus* and which selectively binds Fucα1-2Gal-R ligand motifs; Aleuria aurantia lectin (AAL), which may be derived from *Aleuria aurantia* and which selectively binds Fucα1-2Galβ1-4(Fucα1-3/4)Galβ1-4GlcNAc and R2-GlcNAcβ1-4(Fucα1-6)GlcNAc-R1 ligand motifs; complement receptor 3 (CR3), which may be derived from the plasma membrane of mammalian polymorphonuclear neutrophils and selectively binds polysaccharides such as β-glucans, ligand motifs including N-acetyl D-glucosamine, and the capsular polysaccharide of type III group B *Streptococcus* which includes repeating units of glucose, galactose, N-acetyl D-glucosamine, and N-acetyl neuraminic acid; and *Solanum tuberosum* (STA), which may be derived from potato and which selectively binds (GlcNAc)ₙ. Example animal lectins include Asialoglycoprotein receptor (ASGPR) H1, which selectively binds galactose; Galectin-3 which selectively binds galactose; Sialoadhesin which selectively binds Neu5Ac; Cation-dependent mannose-6-phosphate receptor (CD-MPR), which selectively binds Man6P; and C-reactive protein (CRP), which selectively binds galactose, Gal6P, and galacturonic acid. Con A, LCH, and GNA may be considered "mannose binding lectins." RCA, PHA, AIL, and VVL may be considered "galactose / N-acetylegalactosamine binding lectins." WGA, SNA, and MAL may be considered "sialic acid / N-acetyleglucosamine binding lectins." UEA and AAL may be considered "fucose binding lectins."

Additional example lectins include: Abrus Precatorius Lectin (APA, Jequirity Bean), Aegopodium Podagraria Lectin (APP, Ground Elder), Agaricus bisporus lectin (ASA, mushroom), Limax Flavus Lectin (LFA, Garden Slug); Limulus Polyphemus Lectin (LPA, Horseshoe Crab), Allium Sativum Lectin (ASA, Garlic) Lotus Tetragonolobus Lectin (LOTUS, Asparagus Pea), Anguilla Anguilla Lectin (AAA, Fresh Water Eel), Iris Hybrid Lectin (IRA, Dutch Iris), Lycopersicon Esculentum Lectin (LEA, Tomato) Artocarpus Integrifolia Lectin (Jackfruit), Bauhinia Purpurea Lectin (BPA, Camel's Foot Tree), Maclura Pomifera Lectin (MPA, Osage Orange), Marasmium Oreades Agglutinin Lectin (MOA, Mushroom), Bryonia Dioica Lectin (BDA, White Bryony), Morniga G Lectin (MNA-G, Black Mulberry), Calystega Sepiem Lectin (CALSEPA, Hedge Bindweed Rhizomes), Morniga M Lectin (MNA-M, Black Mulberry), Narcissus Pseudonarcissus Lectin (NPA, Daffodil), Cancer Antennarius Lectin (CCA, California Crab), Phaseolus Lunatus Lectin (LBA, Lima Bean), Caragana Arborescens Lectin (CAA, Pea Tree), Phaseolus Vulgaris Lectin (PHA-E, Red Kidney Bean), Cicer Arietinum Lectin (CPA, Chick Pea), Phaseolus Vulgaris Lectin (PHA-L, Red Kidney Bean) - Colchicum Autumnale Lectin (CA, Meadow Saffron), Phytolacca Americana Lectin (PWM, Pokeweed), Cytisus Sessilifolius Lectin (CSA, Portugal Broom), Pisum Sativum Lectin (PEA, Garden Pea), Datura Stramonium Lectin (DSA, Jimson Weed), Polygonatum Mulitiflorum Lectin (PMA, Common Solomon's Seal), Dioclea Grandiflora Lectin (DGL, Legume), Polyporus Squamosus Lectin (PSL, Mushroom), Erythrina Cristagalli Lectin (ECA, Coral Tree), Euonymus Europaeus Lectin (EEA, Spindle Tree), Tulipa Sp. Lectin (TL, Tulip), Glechoma Hederacea Lectin (GHA, Ground Ivy), Ulex Europaeus Lectin (Gorse), Griffonia Simplicifolia Lectin (GS-I), Urtica Dioica Lectin (UDA, Stinging Nettle), Griffonia Simplicifolia Lectin (GS-II), Vicia Fava Lectin (VFA, Fava Bean), Helix Aspersa Lectin (HAA, Garden Snail), Helix Pomatia Lectin (HPA, Edible Snail), Viscum Album Lectin (VAA, Mistletoe), Hippeastrum Hybrid Lectin (HHA, Amaryllis), and Wisteria Floribunda Lectin (WFA, Japanese Wisteria). Lectins may be engineered or evolved so as to improve binding efficiencies with both natural and unnatural amino acids. In some examples, a lectin may be modified, for example, to improve coupling with one or more sugars, such as by: adding or removing cationic or anionic moieties, by dehydration or dehydrogenation or the reverse processes thereof; or by aminolysis. A "nonlectin protein" is intended to refer to a protein that does not include any lectin.

Example non-natural sugars that Con A may selectively bind include disaccharides in which two sugars are coupled to one another using linkers in a manner such as shown below:

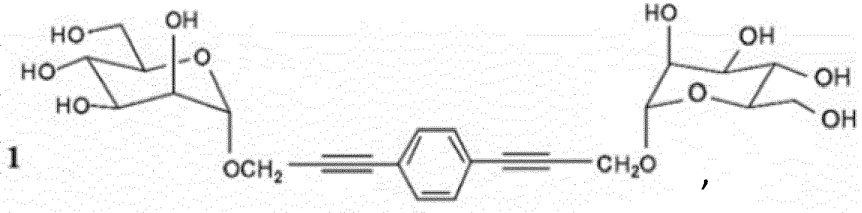

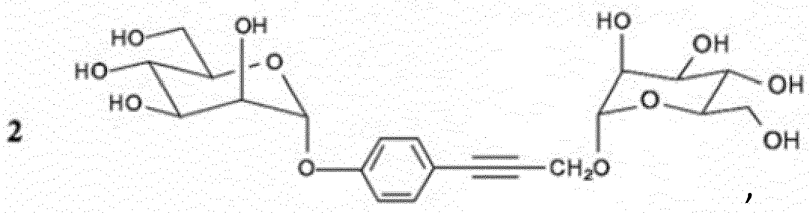

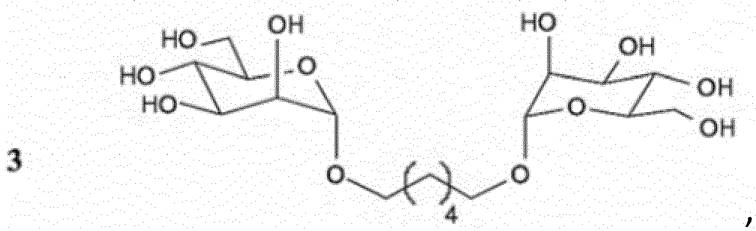

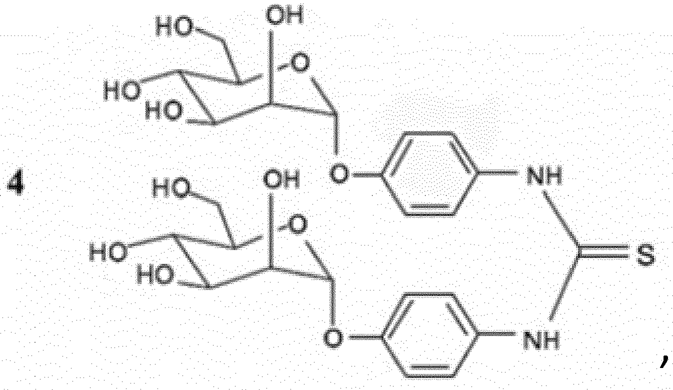

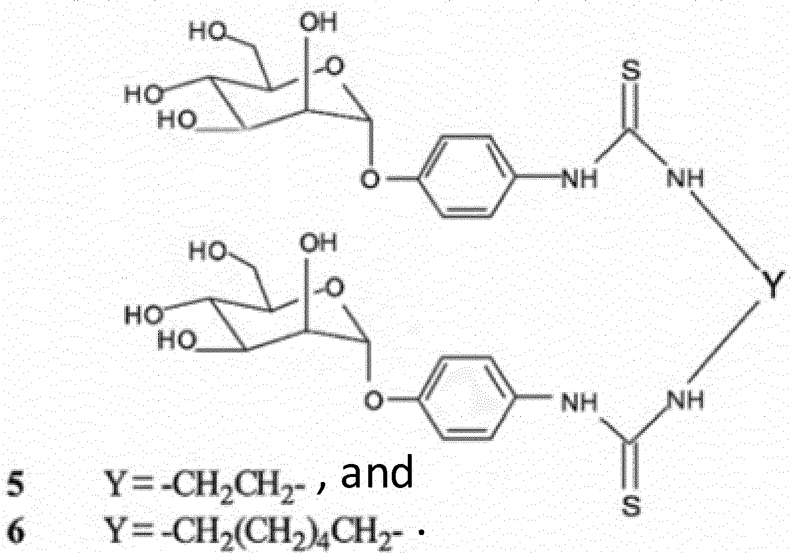

Other example non-natural sugars that Con A may selectively bind include polysaccharides in which more than two sugars are coupled to one another using linkers in a manner such as shown below: 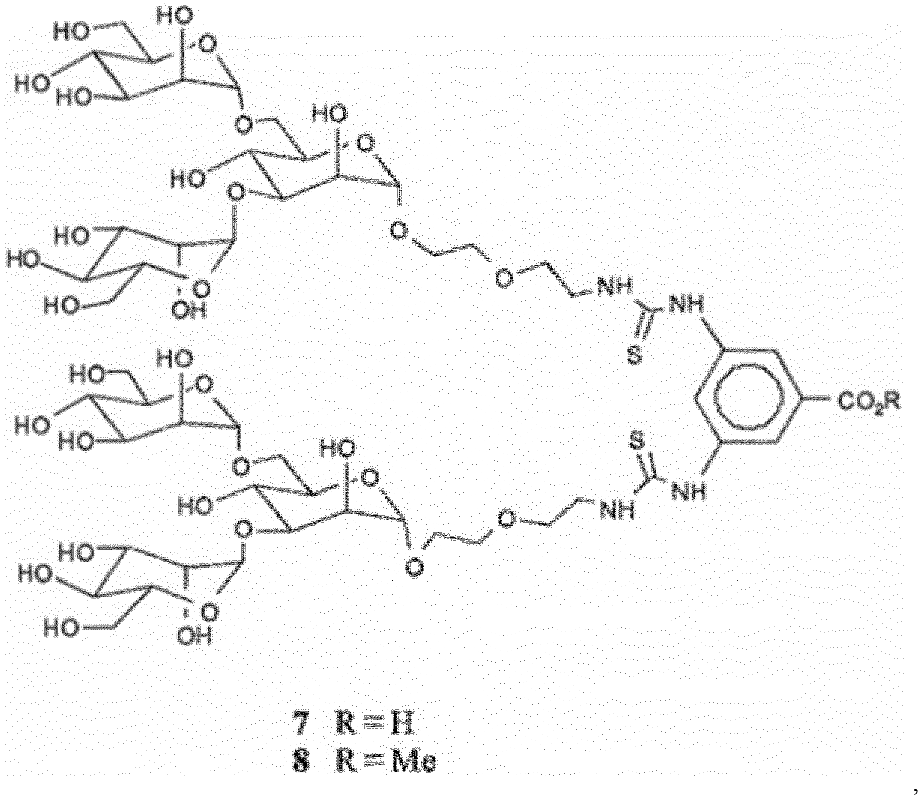 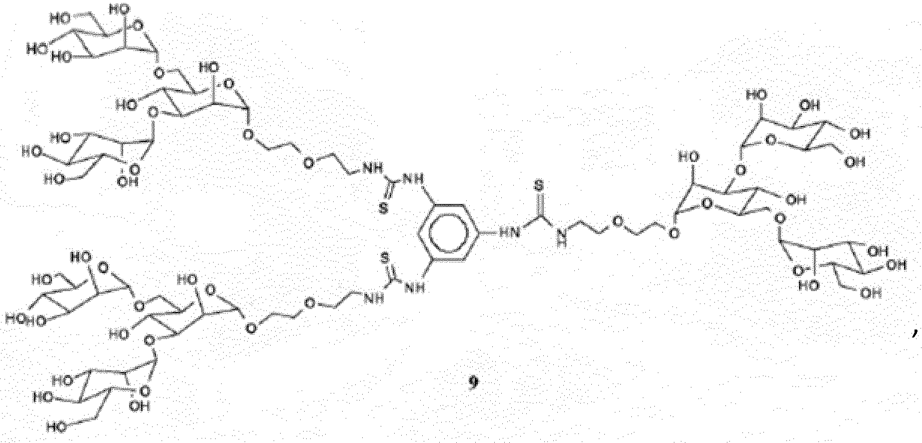 and

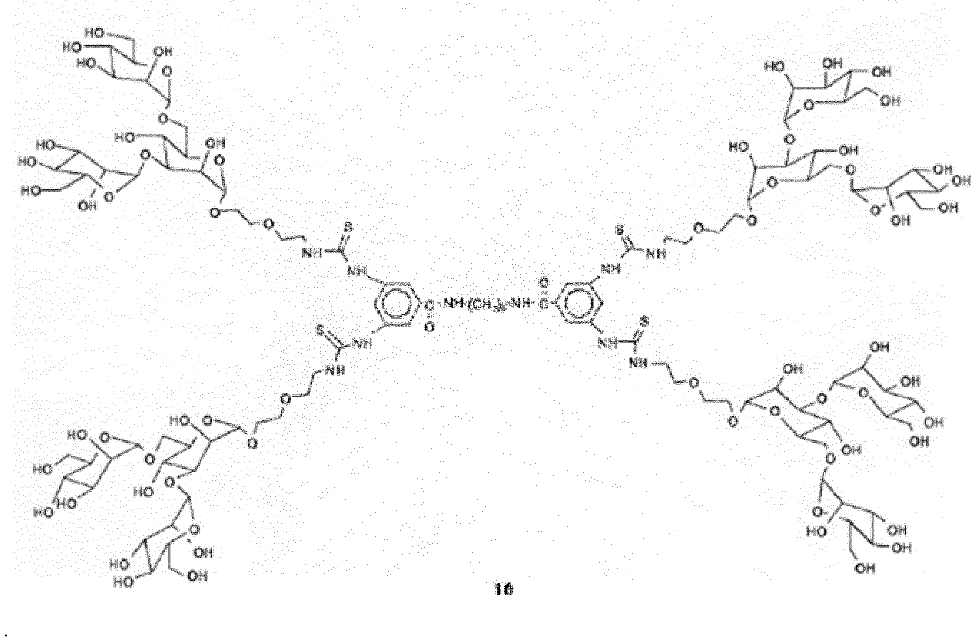

Example non-natural sugars that WGA may selectively bind include the following:

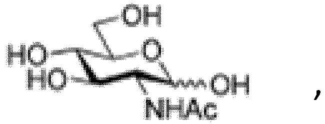

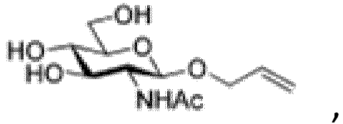

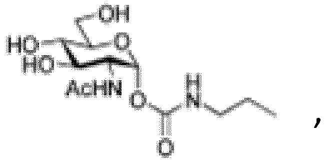

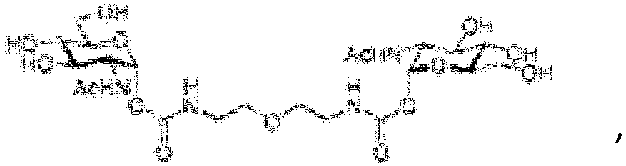

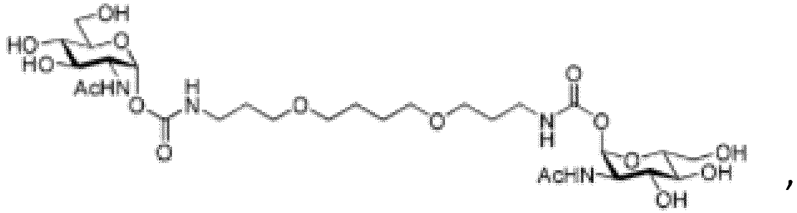

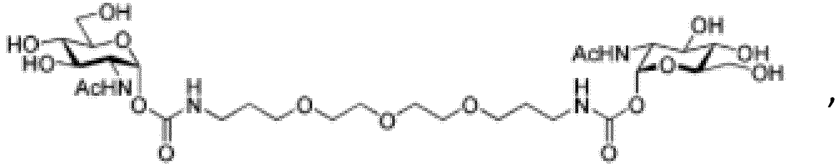

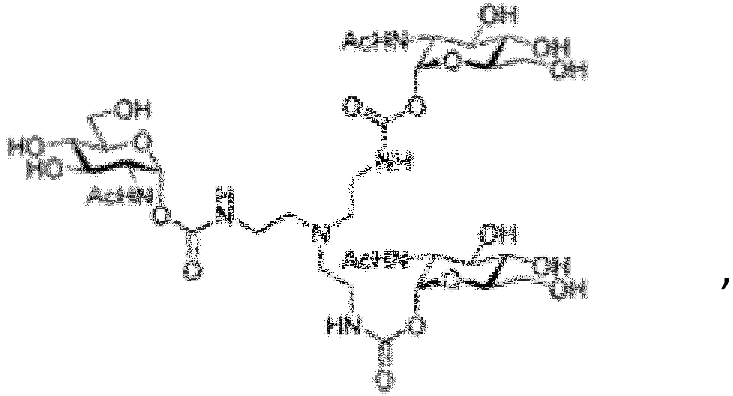

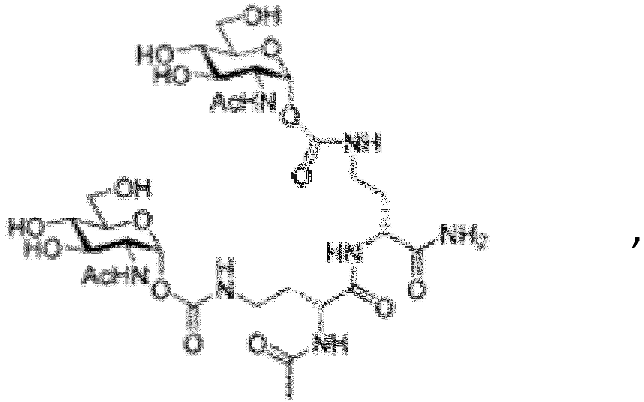

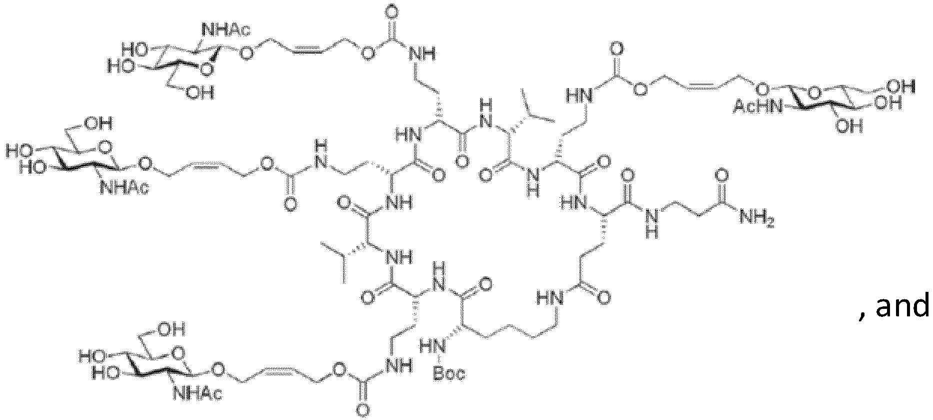

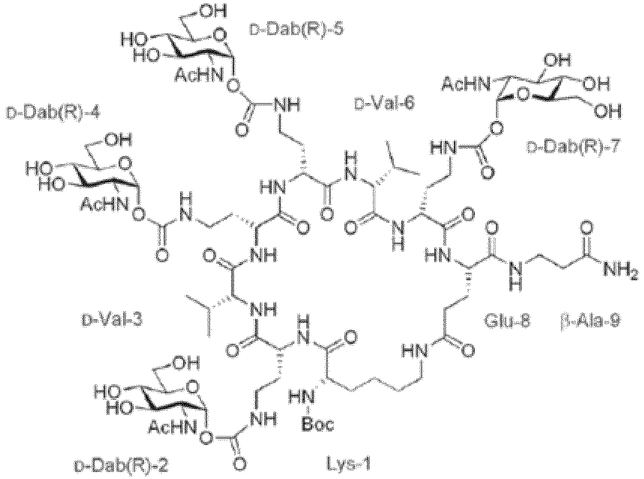

As used herein, "analyte" is intended to mean a chemical element that is desired to be detected. An analyte may be referred to as a "target." Analytes may include nucleotide analytes and non-nucleotide analytes. Analytes for use in the invention comprise a nucleotide. Nucleotide analytes may include one or more nucleotides. Non-nucleotide analytes may include chemical entities that are not nucleotides. An example nucleotide analyte is a DNA analyte, which includes a deoxyribonucleotide or modified deoxyribonucleotide. DNA analytes may include any DNA sequence or feature that may be of interest for detection, such as single nucleotide polymorphisms or DNA methylation. Another example nucleotide analyte is an RNA analyte, which includes a ribonucleotide or modified ribonucleotide. RNA analytes may include any RNA sequence or feature that may be of interest for detection, such as the presence or amount of mRNA or of cDNA. An example non-nucleotide analyte is a protein analyte. A protein includes a sequence of polypeptides that may have a higher order structure, such as a secondary structure, tertiary structure, or quaternary structure. Another example non-nucleotide analyte is a metabolite analyte. A metabolite analyte is a chemical element that is formed or used during metabolism. Additional example analytes include, but are not limited to, carbohydrates such as sugars (e.g., glucose), fatty acids, amino acids, nucleosides, neurotransmitters, phospholipids, and heavy metals. In the present disclosure, analytes may be detected in the context of any suitable application(s), such as analyzing a disease state, analyzing metabolic health, analyzing a microbiome, analyzing drug interaction, analyzing drug response, analyzing toxicity, or analyzing infectious disease. Illustratively, metabolites can include chemical elements that are upregulated or downregulated in response to disease. Nonlimiting examples of analytes include kinases, serine hydrolases, metalloproteases, disease-specific biomarkers such as antigens for specific diseases, and glucose.

As used herein, the term "nucleotide" is intended to mean a molecule that includes a sugar and at least one phosphate group, and optionally also includes a nucleobase. A nucleotide that lacks a nucleobase can be referred to as "abasic." Nucleotides include deoxyribonucleotides, modified deoxyribonucleotides, ribonucleotides, modified ribonucleotides, peptide nucleotides, modified peptide nucleotides, modified phosphate sugar backbone nucleotides, and mixtures thereof. Examples of nucleotides include adenosine monophosphate (AMP), adenosine diphosphate (ADP), adenosine triphosphate (ATP), thymidine monophosphate (TMP), thymidine diphosphate (TDP), thymidine triphosphate (TTP), cytidine monophosphate (CMP), cytidine diphosphate (CDP), cytidine triphosphate (CTP), guanosine monophosphate (GMP), guanosine diphosphate (GDP), guanosine triphosphate (GTP), uridine monophosphate (UMP), uridine diphosphate (UDP), uridine triphosphate (UTP), deoxyadenosine monophosphate (dAMP), deoxyadenosine diphosphate (dADP), deoxyadenosine triphosphate (dATP), deoxythymidine monophosphate (dTMP), deoxythymidine diphosphate (dTDP), deoxythymidine triphosphate (dTTP), deoxycytidine diphosphate (dCDP), deoxycytidine triphosphate (dCTP), deoxyguanosine monophosphate (dGMP), deoxyguanosine diphosphate (dGDP), deoxyguanosine triphosphate (dGTP), deoxyuridine monophosphate (dUMP), deoxyuridine diphosphate (dUDP), deoxyuridine triphosphate (dUTP), dideoxyadenosine triphosphate (ddATP), dideoxythymidine triphosphate (ddTTP), dideoxycytidine triphosphate (ddCTP), dideoxyguanosine triphosphate (ddGTP), dideoxyuridine triphosphate (ddUTP), and the like.

As used herein, the term "nucleotide" also is intended to encompass any nucleotide analogue that may include one or more of a modified nucleobase, sugar and/or phosphate moiety compared to naturally occurring nucleotides. Example modified nucleobases include inosine, xathanine, hypoxathanine, isocytosine, isoguanine, 2-aminopurine, 5-methylcytosine, 5-hydroxymethyl cytosine, 2-aminoadenine, 6-methyl adenine, 6-methyl guanine, 2-propyl guanine, 2-propyl adenine, 2-thiouracil, 2-thiothymine, 2-thiocytosine, 15-halouracil, 15-halocytosine, 5-propynyl uracil, 5-propynyl cytosine, 6-azo uracil, 6-azo cytosine, 6-azo thymine, 5-uracil, 4-thiouracil, 8-halo adenine or guanine, 8-amino adenine or guanine, 8-thiol adenine or guanine, 8-thioalkyl adenine or guanine, 8-hydroxyl adenine or guanine, 5-halo substituted uracil or cytosine, 7-methylguanine, 7-methyladenine, 8-azaguanine, 8-azaadenine, 7-deazaguanine, 7-deazaadenine, 3-deazaguanine, 3-deazaadenine or the like. As is known in the art, certain nucleotide analogues cannot become incorporated into a polynucleotide, for example, nucleotide analogues such as adenosine 5'-phosphosulfate.

As used herein, the term "nucleotide-sugar complex" is intended to mean an element that includes a nucleotide covalently coupled to a sugar. Such covalent coupling may be via a "linker" which itself is neither a nucleotide nor a sugar. Examples of linkers include, but are not limited to, polyethylene glycol (PEG, e.g., PEG₁₋₄₈), (CH₂)ₘ alkyl chain, and polypeptide chains. Examples of covalent couplings that may be used to couple a nucleotide to a sugar include includes amine-NHS ester, amine-imidoester, amine-pentofluorophenyl ester, amine-hydroxymethyl phosphine, carboxyl-carbodiimide, thiol-maleimide, thiol-haloacetyl, thiol-pyridyl disulfide, thiol-thiosulfonate, thiol-vinyl sulfone, aldehyde-hydrazide, aldehyde-alkoxyamine, hydroxy-isocyanate, azide-alkyne, azide-phosphine, transcyclooctene-tetrazine, norbornene-tetrazine, azide-cyclooctyne, and azide-norbornene.

As used herein, the term "polynucleotide" refers to a molecule that includes a sequence of nucleotides that are bonded to one another. A polynucleotide is one nonlimiting example of a polymer. Examples of polynucleotides include deoxyribonucleic acid (DNA), ribonucleic acid (RNA), and analogues thereof. A polynucleotide can be a single stranded sequence of nucleotides, such as RNA or single stranded DNA, a double stranded sequence of nucleotides, such as double stranded DNA or double stranded RNA, or can include a mixture of a single stranded and double stranded sequences of nucleotides. Double stranded DNA (dsDNA) includes genomic DNA, and PCR and amplification products. Single stranded DNA (ssDNA) can be converted to dsDNA and vice-versa. Polynucleotides can include non-naturally occurring DNA, such as enantiomeric DNA. The precise sequence of nucleotides in a polynucleotide can be known or unknown. The following are example examples of polynucleotides: a gene or gene fragment (for example, a probe, primer, expressed sequence tag (EST) or serial analysis of gene expression (SAGE) tag), genomic DNA, genomic DNA fragment, exon, intron, messenger RNA (mRNA), transfer RNA, ribosomal RNA, ribozyme, cDNA, recombinant polynucleotide, synthetic polynucleotide, branched polynucleotide, plasmid, vector, isolated DNA of any sequence, isolated RNA of any sequence, nucleic acid probe, primer or amplified copy of any of the foregoing.

As used herein, "polynucleotide" and "nucleic acid", may be used interchangeably, and can refer to a polymeric form of nucleotides of any length, such as either ribonucleotides or deoxyribonucleotides. Thus, this term includes single-, double-, or multi-stranded DNA or RNA. The term polynucleotide also refers to both double and single-stranded molecules. Examples of polynucleotides include a gene or gene fragment, genomic DNA, genomic DNA fragment, exon, intron, messenger RNA (mRNA), transfer RNA, ribosomal RNA, non-coding RNA (ncRNA) such as PIWI-interacting RNA (piRNA), small interfering RNA (siRNA), and long non-coding RNA (lncRNA), small hairpin (shRNA), small nuclear RNA (snRNA), micro RNA (miRNA), small nucleolar RNA (snoRNA) and viral RNA, ribozyme, cDNA, recombinant polynucleotide, branched polynucleotide, plasmid, vector, isolated DNA of any sequence, isolated RNA of any sequence, nucleic acid probe, primer or amplified copy of any of the foregoing. A polynucleotide can include modified nucleotides, such as methylated nucleotides and nucleotide analogs including nucleotides with non-natural bases, nucleotides with modified natural bases such as aza- or deaza-purines. In some examples, a polynucleotide can be composed of a specific sequence of four nucleotide bases: adenine (A); cytosine (C); guanine (G); and thymine (T). Uracil (U) can also be present, for example, as a natural replacement for thymine when the polynucleotide is RNA. Uracil can also be used in DNA. Thus, the term 'sequence' refers to the alphabetical representation of a polynucleotide or any nucleic acid molecule, including natural and non-natural bases.

As used herein, "target nucleic acid" or grammatical equivalent thereof can refer to nucleic acid molecules or sequences that it is desired to identify, sequence, analyze and/or further manipulate. In some examples, a target nucleic acid can include a single nucleotide polymorphism (SNP) to be identified. In some examples, a SNP can be identified by hybridizing a probe to the target nucleic acid, and extending the probe. In some examples, the extended probe can be detected by hybridizing the extended probe to a capture probe.

As used herein, "hybridize" is intended to mean noncovalently attaching a first polynucleotide to a second polynucleotide along the lengths of those polynucleotides via specific hydrogen bonding pairing of nucleotide bases. The strength of the attachment between the first and second polynucleotides increases with the length and complementarity between the sequences of monomer units within those polymers. For example, the strength of the attachment between a first polynucleotide and a second polynucleotide increases with the complementarity between the sequences of nucleotides within those polynucleotides, and with the length of that complementarity. By "temporarily hybridized" it is meant that polymer sequences are hybridized to each other at a first time, and dehybridized from one another at a second time.

For example, as used herein, "hybridization", "hybridizing" or grammatical equivalent thereof, can refer to a reaction in which one or more polynucleotides react to form a complex that is formed at least in part via hydrogen bonding between the bases of the nucleotide residues. The hydrogen bonding can occur by Watson-Crick base pairing, Hoogstein binding, or in any other sequence-specific manner. The complex can have two strands forming a duplex structure, three or more strands forming a multi-stranded complex, a single self-hybridizing strand, or any combination of thereof. The strands can also be cross-linked or otherwise joined by forces in addition to hydrogen bonding.

As used herein, a "polymerase" is intended to mean an enzyme having an active site that assembles polynucleotides by polymerizing nucleotides into polynucleotides. A polymerase can bind a primed single stranded polynucleotide template, and can sequentially add nucleotides to the growing primer to form a polynucleotide having a sequence that is complementary to that of the template.

As used herein, the term "primer" is defined as a polynucleotide having a single strand with a free 3' OH group. A primer can also have a modification at the 5' terminus to allow a coupling reaction or to couple the primer to another moiety. The primer length can be any number of bases long and can include a variety of non-natural nucleotides. A primer can be blocked at the 3' end to inhibit polymerization until the block is removed.

As used herein, "extending", "extension" or any grammatical equivalents thereof can refer to the addition of dNTPs or ddNTPs to a primer, polynucleotide or other nucleic acid molecule by an extension enzyme such as a polymerase, or ligase.

As used herein, the term "detectable moiety" is intended to mean a structure that is coupled to an element and based upon which the presence of an element can be detected. A detectable moiety may include a moiety to which a fluorophore, or other element that can be detected, may be coupled directly or indirectly. For example, the element's label may include a sugar, and the fluorophore, or other element that may be detected, may be coupled to a lectin that becomes coupled indirectly to the element by being coupled to the sugar. A non-limiting example of a detectable moiety is a fluorophore, which may be detected using optical circuitry. Another non-limiting example of a detectable moiety is a structure that couples to an element through which electrical current flows and that detectably changes an electrical characteristic of that element.

As used herein, the term "substrate" refers to a material used as a support for compositions described herein. Example substrate materials may include glass, silica, plastic, quartz, metal, metal oxide, organo-silicate (e.g., polyhedral organic silsesquioxanes (POSS)), polyacrylates, tantalum oxide, complementary metal oxide semiconductor (CMOS), or combinations thereof. An example of POSS can be that described in Kehagias et al., Microelectronic Engineering 86 (2009), pp. 776-778. In some examples, substrates used in the present application include silica-based substrates, such as glass, fused silica, or other silica-containing material. In some examples, silica-based substrates can include silicon, silicon dioxide, silicon nitride, or silicone hydride. In some examples, substrates used in the present application include plastic materials or components such as polyethylene, polystyrene, poly(vinyl chloride), polypropylene, nylons, polyesters, polycarbonates, and poly(methyl methacrylate). Example plastics materials include poly(methyl methacrylate), polystyrene, and cyclic olefin polymer substrates. In some examples, the substrate is or includes a silica-based material or plastic material or a combination thereof. In particular examples, the substrate has at least one surface including glass or a silicon-based polymer. In some examples, the substrates can include a metal. In some such examples, the metal is gold. In some examples, the substrate has at least one surface including a metal oxide. In one example, the surface includes a tantalum oxide or tin oxide. Acrylamides, enones, or acrylates may also be utilized as a substrate material or component. Other substrate materials can include, but are not limited to gallium arsenide, indium phosphide, aluminum, ceramics, polyimide, quartz, resins, polymers and copolymers. In some examples, the substrate and/or the substrate surface can be, or include, quartz. In some other examples, the substrate and/or the substrate surface can be, or include, semiconductor, such as GaAs or ITO. The foregoing lists are intended to be illustrative of, but not limiting to the present application. Substrates can include a single material or a plurality of different materials. Substrates can be composites or laminates. In some examples, the substrate includes an organo-silicate material.

Substrates can be flat, round, spherical, rod-shaped, or any other suitable shape. Substrates may be rigid or flexible. In some examples, a substrate is a bead or a flow cell, or a bead located in a flow cell.

Substrates can be non-patterned, textured, or patterned on one or more surfaces of the substrate. In some examples, the substrate is patterned. Such patterns may include posts, pads, wells, ridges, channels, or other three-dimensional concave or convex structures. Patterns may be regular or irregular across the surface of the substrate. Patterns can be formed, for example, by nanoimprint lithography or by use of metal pads that form features on non-metallic surfaces, for example.

In some examples, a substrate described herein forms at least part of a flow cell or is located in or coupled to a flow cell. Flow cells may include a flow chamber that is divided into a plurality of lanes or a plurality of sectors. Example flow cells and substrates for manufacture of flow cells that can be used in methods and compositions set forth herein include, but are not limited to, those commercially available from Illumina, Inc. (San Diego, CA). Beads may be located in a flow cell.

As used herein, "surface" can refer to a part of a substrate or support structure that is accessible to contact with reagents, beads or analytes. The surface can be substantially flat or planar. Alternatively, the surface can be rounded or contoured. Example contours that can be included on a surface are wells, depressions, pillars, ridges, channels or the like. Example materials that can be used as a substrate or support structure include glass such as modified or functionalized glass; plastic such as acrylic, polystyrene or a copolymer of styrene and another material, polypropylene, polyethylene, polybutylene, polyurethane or TEFLON; polysaccharides or cross-linked polysaccharides such as agarose or Sepharose; nylon; nitrocellulose; resin; silica or silica-based materials including silicon and modified silicon; carbon-fibre; metal; inorganic glass; optical fibre bundle, or a variety of other polymers. A single material or mixture of several different materials can form a surface useful in certain examples. In some examples, a surface comprises wells. In some examples, a support structure can include one or more layers. Example support structures can include a chip, a film, a multi-well plate, and a flow-cell.

As used herein, "bead" can refer to a small body made of a solid material. The material of the bead may be rigid or semi-rigid. The body can have a shape characterized, for example, as a sphere, oval, microsphere, or other recognized particle shape whether having regular or irregular dimensions. In some examples, a bead or a plurality of beads can comprise a surface. Example materials that are useful for beads include glass such as modified or functionalized glass; plastic such as acrylic, polystyrene or a copolymer of styrene and another material, polypropylene, polyethylene, polybutylene, polyurethane or TEFLON; polysaccharides or cross-linked polysaccharides such as agarose or Sepharose; nylon; nitrocellulose; resin; silica or silica-based materials including silicon and modified silicon; carbon-fiber; metal; inorganic glass; or a variety of other polymers. Example beads include controlled pore glass beads, paramagnetic beads, thoria sol, Sepharose beads, nanocrystals and others known in the art. Beads can be made of biological or non-biological materials. Magnetic beads are particularly useful due to the ease of manipulation of magnetic beads using magnets at various processes of the methods described herein. Beads used in certain examples can have a diameter, width or length from about 5.0 nm to about 100 µm, e.g., from about 10 nm to about 100 µm, e.g., from about 50 nm to about 50 µm, e.g., from about 100 nm to about 500 nm. In some examples, beads used in certain examples can have a diameter, width or length less than about 100 µm, 50 µm, 10 µm, 5 µm, 1 µm, 0.5 µm, 100 nm, 50 nm, 10 nm, 5 nm, 1 nm, 0.5 nm, 100 pm, or any diameter, width or length within a range of any two of the foregoing diameters, widths or lengths. Bead size can be selected to have reduced size, and hence get more features per unit area, whilst maintaining sufficient signal (template copies per feature) in order to analyze the features.

In some examples, polynucleotides, such as capture probes or codes can be coupled to beads. In some examples, the beads can be distributed into wells on the surface of a substrate, such as a flow cell. Example bead arrays that can be used in certain examples include randomly ordered BEADARRAY technology (Illumina Inc., San Diego CA).

As used herein, a "polymer" refers to a molecule including a chain of many subunits that are coupled to one another and that may be referred to as monomers. The subunits may repeat, or may differ from one another. Polymers can be biological or synthetic polymers. Example biological polymers that suitably can be included within a label include polynucleotides, polypeptides, polysaccharides, polynucleotide analogs, and polypeptide analogs. Example polynucleotides and polynucleotide analogs include DNA, enantiomeric DNA, RNA, PNA (peptide-nucleic acid), morpholinos, and LNA (locked nucleic acid). Polymers may include spacer phosphoramidites, which may be coupled to polynucleotides but which lack nucleobases, such as commercially available from Glen Research (Sterling, VA). Example synthetic polypeptides can include charged or neutral amino acids as well as hydrophilic and hydrophobic residues. Example synthetic polymers include PEG (polyethylene glycol), PPG (polypropylene glycol), PVA (polyvinyl alcohol), PE (polyethylene), LDPE (low density polyethylene), HDPE (high density polyethylene), polypropylene, PVC (polyvinyl chloride), PS (polystyrene), NYLON (aliphatic polyamides), TEFLON^{®} (tetrafluoroethylene), thermoplastic polyurethanes, polyaldehydes, polyolefins, poly(ethylene oxides), poly(ω-alkenoic acid esters), poly(alkyl methacrylates), and other polymeric chemical and biological linkers..

As used herein, a "type" is intended to mean having a distinguishable characteristic. As such, an element being of a "different" type than another element means that one element is has at least one characteristic that is distinguishable from at least one characteristic of the other element such that the elements are distinguishable from one another in a manner other than occupying different physical spaces than one another. In comparison, elements that are the same type as one another may occupy different physical spaces than one another, but are otherwise indistinguishable from one another because they both have the same characteristics as one another. In comparison, two elements that are different types than one another may occupy different physical spaces than one another, and also may be distinguishable from one another because at least one of their characteristics is different. Illustratively, several different types of nucleotides are described herein, such as ddATP, ddTTP, dddGTP, dUTP, and ddCTP which have different bases than one another, as well as nucleotide analogues having different modifications than one another such as different sugars, different phosphates, and the like. Any two given nucleotides may occupy different physical spaces than one another, and may be the same type as one another (e.g., both may include ddATP) or may be different types than one another (e.g., one may include ddATP and the other may include ddTTP). Analogously, several different types of sugars are described herein, such as different types of monosaccharides (e.g., mannose, glucose, fructose, galactose, ribose, and deoxyribose), different types of disaccharides (e.g., lactose, sucrose, and maltose), and other sugar types such as N-acetyl-glucosamine (GlcNAc)ₙ, N-acetyl-neuraminic acid (Neu5Ac), N-acetyl-galactosamine (GalNAc), Galβ3GalNAcα, GalNAcα3GalNAc, Man6P, and galacturonic acid. Any two given sugars may occupy different physical spaces than one another, and may be the same type as one another (e.g., both may include glucose) or may be different types than one another (e.g., one may include glucose and the other may include galacturonic acid). Analogously, several different types of lectins are described herein that are selective to respective types of sugars. Any two given lectins may occupy different physical spaces than one another, and may be the same type as one another (e.g., both may include ConA) or may be different types than one another (e.g., one may include ConA and the other may include WGA). Analogously, different types of fluorophores are described herein. Any two given fluorophores may occupy different physical spaces than one another, and may be the same type as one another (e.g., both may fluoresce at the same wavelength) or may be different types than one another (e.g., may fluoresce at different wavelengths).

### Example systems and methods for detecting elements using sugar-lectin couplings

Technologies that use fluorescent labels to detect analytes, such as nucleotides, may be limited by signal intensity, uniformity, and linear dynamic range. These include sequencing-by-synthesis applications where low signal intensity may become an issue, particularly as feature sizes in flow cells become smaller, resulting in a decrease in the number of sequencing templates per cluster. Another example is genotyping array platforms where detection of a low number of molecules captured per bead would benefit from enhancement in signal relative to a single fluorescent labeling event. Provided herein are several example methods for detecting elements, such as analytes, by coupling detectable moieties (such as, but not limited to, fluorophores) to the elements using sugar-lectin couplings. It will be appreciated that the present methods suitably may be adapted to couple any detectable moiety to any desired element, for detection in any suitable system.

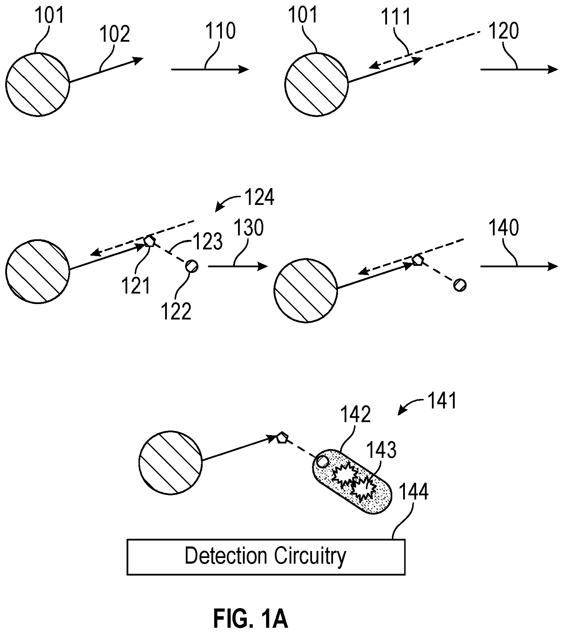

FIGS. 1A-1B schematically illustrate example process flows for detecting an element using a sugar-lectin coupling. In the nonlimiting example illustrated in FIG. 1A, the element to be detected includes an analyte, such as a nucleotide that may be used in a single-base addition process to characterize an oligonucleotide, e.g., to determine the presence or absence of an oligonucleotide, or to detect and determine the identity of a particular nucleotide in the sequence of an oligonucleotide. Illustratively, first oligonucleotide 102 is coupled to substrate 101, such as a bead as is illustrated in FIG. 1A or as a planar substrate or a feature on or in a planar substrate (not specifically illustrated). First oligonucleotide 102 may, for example, include a sequence that is complementary to a sequence that it is desired to characterize. At process 110 illustrated in FIG. 1A, second oligonucleotide 111 hybridizes with first oligonucleotide 102 and thereby becomes coupled to substrate 101. At process 120, nucleotide-sugar complex 124 may be coupled to substrate 101, and it is desired to detect the presence of the nucleotide or both detect and determine the identity of the nucleotide. As illustrated in FIG. 1A, nucleotide-sugar complex 124 may include nucleotide 121 coupled to sugar 122, e.g., via linker 123. At process 120, nucleotide 121 may be incorporated by a polymerase (not specifically illustrated) into first oligonucleotide 102 using at least the sequence of second oligonucleotide 111, and may be used to characterize second oligonucleotide 111. For example, the presence of nucleotide 121 may be used to determine the presence of second oligonucleotide 111, and the identity of nucleotide 121 may be used to determine the identity within second oligonucleotide 111 of the nucleotide that is complementary to nucleotide 121.

A sugar-lectin coupling may be used to detect nucleotide 121, and in some examples may be used to identify nucleotide 121. For example, at process 130 illustrated in FIG. 1A, second oligonucleotide 111 may be dehybridized from first oligonucleotide 102. At process 140 illustrated in FIG. 1A, lectin 142 may be coupled to sugar 122, and nucleotide 121 detected using at least detection of lectin 142. For example, system 141 illustrated in FIG. 1A may include a composition including substrate 101, nucleotide 121 coupled to the substrate (e.g., via first oligonucleotide 102), sugar 122, and lectin 142, as well as detection circuitry 144 to detect nucleotide 121 using at least detection of the lectin 142. Illustratively, fluorophore 143 may be coupled to lectin 142, and detection circuitry 144 may include an optical sensor to detect lectin 142 using at least detection of fluorescence from fluorophore 143.

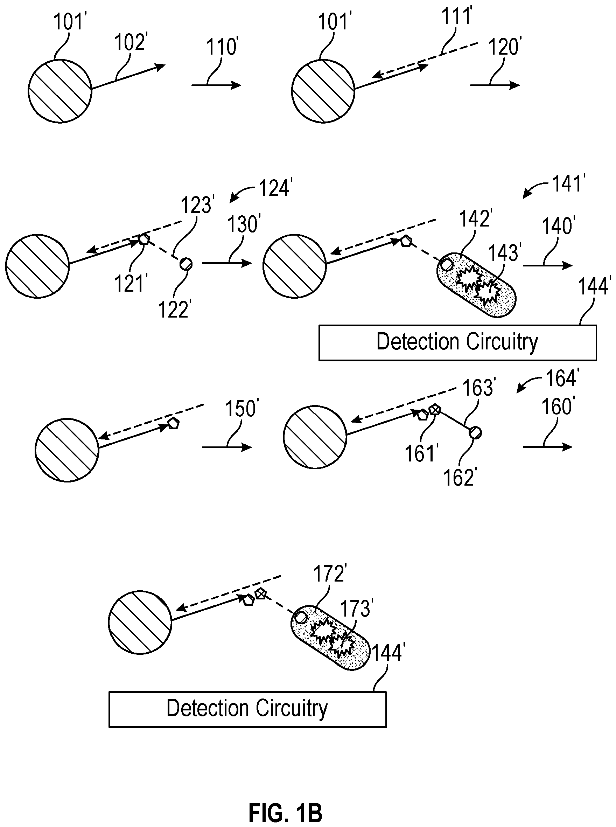

In the nonlimiting example illustrated in FIG. 1B, the element to be detected includes an analyte, such as a nucleotide that may be used in sequencing-by-synthesis process to determine multiple nucleotides in the sequence of an oligonucleotide. Illustratively, first oligonucleotide 102' is coupled to substrate 101', such as a bead as is illustrated in FIG. 1B or as a planar substrate or a feature on or in a planar substrate (not specifically illustrated). First oligonucleotide 102' may, for example, include a sequence that is complementary to a sequence that it is desired to characterize, e.g., to sequence using a sequencing-by-synthesis process. At process 110' illustrated in FIG. 1B, second oligonucleotide 111' hybridizes with first oligonucleotide 102' and thereby becomes coupled to substrate 101'. At process 120', first nucleotide-sugar complex 124' may be coupled to substrate 101', and it is desired to detect the identity of the first nucleotide. As illustrated in FIG. 1B, first nucleotide-sugar complex 124' may include first nucleotide 121' coupled to first sugar 122', e.g., via first linker 123'. At process 120', first nucleotide 121' may be incorporated by a polymerase (not specifically illustrated) so as to extend first oligonucleotide 102' using at least the sequence of second oligonucleotide 111', and may be used to characterize second oligonucleotide 111'. For example, the identity of first nucleotide 121' may be used to determine the sequence of second oligonucleotide 111' at a particular location within the second oligonucleotide.

A sugar-lectin coupling may be used to detect and identify first nucleotide 121'. For example, at process 130' illustrated in FIG. 1B, first lectin 142' may be coupled to first sugar 122', and first nucleotide 121' detected and identified using at least detection of first lectin 142'. For example, system 141' illustrated in FIG. 1B may include a composition including substrate 101', first nucleotide 121' coupled to the substrate (e.g., via first oligonucleotide 102'), first sugar 122', and first lectin 142', as well as detection circuitry 144' to detect and identify first nucleotide 121' using at least detection of the first lectin 142'. Illustratively, first fluorophore 143' may be coupled to first lectin 142', and detection circuitry 144' may include an optical sensor to detect first lectin 142' using at least detection of fluorescence from first fluorophore 143'. After first lectin 142' is detected, at process 140' first linker 123', first sugar 122', and first lectin 142' may be cleaved from first nucleotide 121' so as to prepare first oligonucleotide 102', as extended using first nucleotide 121', for addition of one or more additional nucleotides using at least the sequence of second oligonucleotide 111'.

For example, at process 150' illustrated in FIG. 1B, second nucleotide-sugar complex 164' may be coupled to substrate 101', and it is desired to detect the identity of the second nucleotide. As illustrated in FIG. 1B, second nucleotide-sugar complex 164' may include second nucleotide 161' coupled to second sugar 162', e.g., via second linker 163'. At process 160', second nucleotide 161' may be incorporated by a polymerase (not specifically illustrated) so as to extend first oligonucleotide 102' using at least the sequence of second oligonucleotide 111', and may be used to characterize second oligonucleotide 111'. For example, the identity of second nucleotide 161' may be used to determine the sequence of second oligonucleotide 111' at a particular location within the second oligonucleotide.

A sugar-lectin coupling may be used to detect and identify second nucleotide 161'. For example, at process 160' illustrated in FIG. 1B, second lectin 172' may be coupled to second sugar 162', and second nucleotide 161' detected and identified using at least detection of second lectin 172'. For example, detection circuitry 144' further may be to detect and identify second nucleotide 161' using at least detection of the second lectin 172'. Illustratively, second fluorophore 173' may be coupled to second lectin 172', and detection circuitry 144' may include an optical sensor to detect second lectin 142' using at least detection of fluorescence from second fluorophore 173'. For example, second sugar 162' may be a different type of sugar than first sugar 122', second lectin 172' may be a different type of lectin than first lectin 142'(e.g., may selectively bind to second sugar 162' and may not bind to first sugar 122') , and second fluorophore 173' may be a different type of fluorophore than first fluorophore 143'. As such, a different type of fluorophore may become coupled (via second lectin 172') to second nucleotide 161' than is coupled (via first lectin 142') to first nucleotide 121', and the different types of fluorophores may be optically distinguishable from one another, thus permitting the first and second nucleotides to be distinguished from one another. After second lectin 172' is detected, second linker 163', second sugar 162', and second lectin 173' are cleaved from second nucleotide 161' so as to prepare first oligonucleotide 102', as extended using first nucleotide 121' and second nucleotide 161', for addition of one or more additional nucleotides using at least the sequence of second oligonucleotide 111'.

In examples such as described with reference to FIGS. 1A-1B, it should be appreciated that a lectin may include or be coupled to any suitable moiety that is detectable using detection circuitry, e.g., is not limited to use with fluorophores and optical detection circuitry. Furthermore, it should be appreciated that any suitable element, e.g., analyte, may be coupled to a sugar to which a lectin may be coupled so as to detect the element. As such, the elements that may be detected using the present sugar-lectin couplings are not limited to the specific analytes (nucleotides) described with reference to in the non-limiting examples of FIGS. 1A-1B.

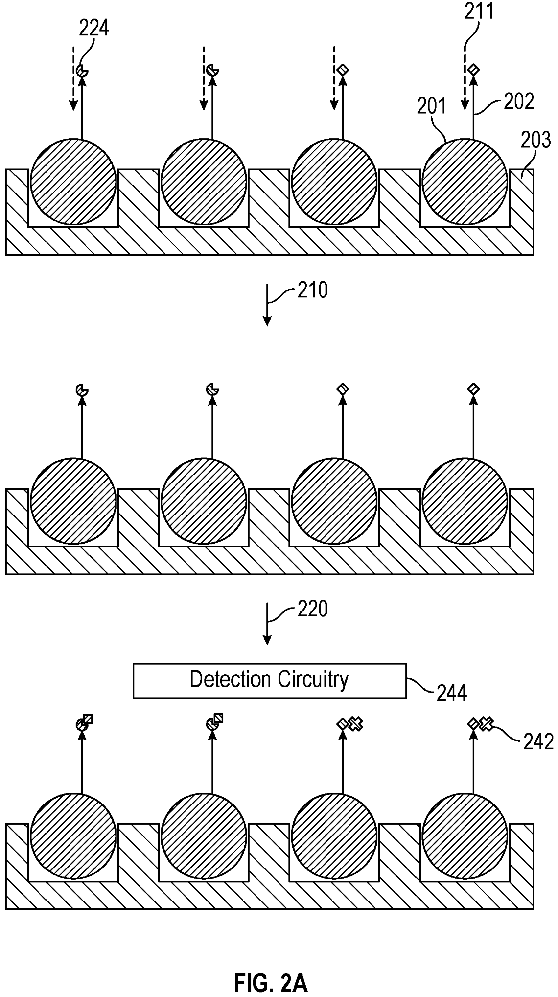

Any suitable, and different, types of sugars respectively may be coupled to different elements than one another, and lectins that are selective to those types of sugars may be used to detect the different elements. For example, FIGS. 2A-2B schematically illustrate additional example process flows for detecting elements using sugar-lectin couplings.

In the nonlimiting example illustrated in FIG. 2A, the elements to be detected include analytes, such as nucleotides that may be used in a single-base addition process to characterize oligonucleotides, e.g., to determine the presence or absence of oligonucleotides, or to detect and determine the identity of particular nucleotides in the sequences of oligonucleotides. Illustratively, a plurality of first oligonucleotides 202 are coupled to respective substrates 201 (a single such first oligonucleotide and a single such substrate being labeled in FIG. 2A). Substrates 201 may include beads that may be located within respective wells 203 in a flowcell, as is illustrated in FIG. 2A, or may include respective features on or in a planar substrate (not specifically illustrated). Each of the first oligonucleotides 202 may, for example, include a sequence that is complementary to a sequence that it is desired to characterize. The sequences of the first oligonucleotides 202 may be different than one another, or may be the same as one another. As illustrated in FIG. 2A, second oligonucleotides 211 may be hybridized with respective first oligonucleotides 202 and thereby are coupled to respective substrates 201. As such, second oligonucleotides 211 respectively may have sequences that are complementary to the sequences of the first oligonucleotides 202 to which they are hybridized.

In a manner similar to that described with reference to FIG. 1A, nucleotide-sugar complexes 224 may be coupled to respective substrates 201, and it is desired to detect the presence of the nucleotides or both detect and determine the identity of the nucleotides. Illustratively, a solution may be flowed over the substrates 201 that may include a plurality of element-sugar complexes that include different element types coupled to different sugar types than one another, e.g., a plurality of nucleotide-sugar complexes that include different nucleotide types respectively coupled to different sugar types than one another. For example, nucleotide-sugar complexes 224 may include respective nucleotides coupled to sugars, e.g., via respective linkers. Illustratively, the nucleotide-sugar complexes may include one or more ddNTP-sugar complexes, such as a ddCTP-sugarl complex, a ddGTP-sugar2 complex, a ddATP-sugar3 complex, and a ddTTP-sugar4 (or ddUTP-sugar4) complex, where sugar1, sugar2, sugar3, and sugar4 include different sugar types than one another. In a manner similar to that described with reference to FIG. 1A, the nucleotides of the nucleotide-sugar complexes 224 may be incorporated by polymerases (not specifically illustrated) into respective first oligonucleotides 202 using at least the sequence of respective second oligonucleotides 211, and may be used to characterize second oligonucleotides 211. For example, the presence of the nucleotides of the nucleotide-sugar complexes 224 may be used to determine the presence of respective second oligonucleotides 211, and the identities of the nucleotides of the nucleotide-sugar complexes 224 may be used to determine the identities within respective second oligonucleotides 211 of the nucleotides that are complementary to those nucleotides.

Sugar-lectin couplings may be used to detect the nucleotides of the nucleotide-sugar complexes 224, and in some examples may be used to identify the nucleotides of the nucleotide-sugar complexes 224. For example, at process 210 illustrated in FIG. 2A, second oligonucleotides 211 may be dehybridized from first oligonucleotides 202. At process 220 illustrated in FIG. 2A, lectins 242 may be coupled to the sugars of the nucleotide-sugar complexes 224, and the nucleotides of those nucleotide-sugar complexes 224 detected using at least detection of the lectins 242. For example, the system illustrated in FIG. 2A may include a composition including substrates 201, nucleotide-sugar complexes 224 coupled to respective substrates (e.g., via respective first oligonucleotides 202), and lectins 242, as well as detection circuitry 244 to detect the nucleotides using at least detection of the respective lectins 242. Illustratively, fluorophores may be coupled to respective lectins 242, and detection circuitry 244 may include an optical sensor to detect lectins 242 using at least detection of fluorescence from the fluorophores. For example, lectins 242 may include a Lectin1-Dye1 complex, a Lectin2-Dye2 complex, a Lectin3-Dye3 complex, and a Lectin4-Dye4 complex, where Dye1, Dye2, Dye3, and Dye4 include different fluorophores emitting different wavelengths than one another, and where Lectin1 selectively binds sugar1 and not any of the other sugar types (e.g., does not bind sugar2, sugar3, and sugar4), Lectin2 selectively binds sugar2 and not any of the other sugar types, Lectin3 selectively binds sugar3 and not any of the other sugar types, and Lectin4 selectively binds sugar4 and not any of the other sugar types. That is, Lectin1, Lectin2, Lectin3, and Lectin4 may include different lectin types than one another. Using at least the selective binding of the different lectins 242 to the different sugar types of the nucleotide-sugar complexes 224, and the different fluorophores (or other detectable moieties) coupled to those lectins, the different nucleotides of those nucleotide-sugar complexes 224 may be detected and identified using detection circuitry 244.

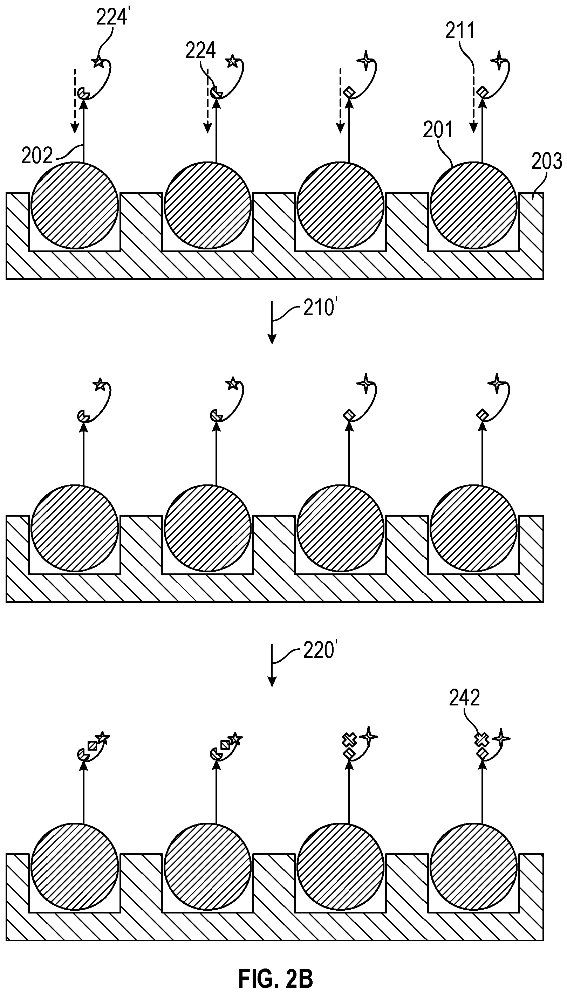

In the nonlimiting example illustrated in FIG. 2B, the elements to be detected include analytes, such as nucleotides that may be used in a single-base addition process to characterize oligonucleotides, e.g., to determine the presence or absence of oligonucleotides, or to detect and determine the identity of particular nucleotides in the sequences of oligonucleotides. Similarly as described with reference to FIG. 2A, in FIG. 2B a plurality of first oligonucleotides 202 are coupled to respective substrates 201, such as beads located within respective wells 203 in a flowcell. Each of the first oligonucleotides 202 may, for example, include a sequence that is complementary to a sequence that it is desired to characterize, a sequence complementary to that of second oligonucleotides 211. In a manner similar to that described with reference to FIGS. 1A and 2A, nucleotide-sugar complexes may be coupled to respective substrates 201, and it is desired to detect the presence of the nucleotides or both detect and determine the identity of the nucleotides. However, while in examples such as described with reference to FIG. 2A the nucleotide-sugar complexes 224 each may include a single sugar for which a respective lectin 242 is selective, in FIG. 2B the nucleotide-sugar complexes each may include a nucleotide and two or more sugars coupled to the nucleotide via respective linkage(s), e.g., may include nucleotide-sugar complex 224 and one or more additional sugars 224'. Lectins 242 may be divalent or multivalent and therefore may selectively bind each of the sugars.

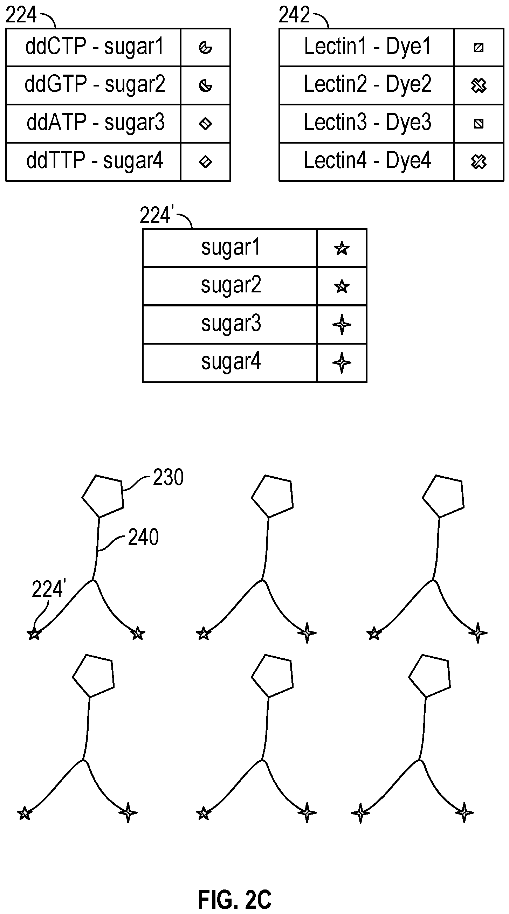

Illustratively, a solution may be flowed over the substrates 201 that may include a plurality of element-sugar complexes that include different element types coupled to different sugar types than one another, e.g., a plurality of nucleotide-sugar complexes that include different nucleotide types each coupled to multiple sugars (e.g., to two or more sugars), where at least one of the sugar types coupled to each of the nucleotides differs from at least one of the sugar types coupled to another of the nucleotides. Illustratively, the nucleotide-sugar complexes may include one or more ddNTP-sugar complexes, such as a complex including ddCTP-sugarl and an additional sugar1, a complex including ddGTP-sugar2 and an additional sugar2, a complex including ddATP-sugar3 and an additional sugar3, and a complex including ddTTP-sugar4 (or ddUTP-sugar4) and an additional sugar4, where sugar1, sugar2, sugar3, and sugar4 include different sugar types. In some examples such as illustrated in FIG. 2B, two of each particular sugar type is included within the respective complex; alternatively, in examples such as described further below with reference to FIG. 2C, one or more of the nucleotide-sugar complexes may include two or more different sugars. In a manner similar to that described with reference to FIG. 1A, the nucleotides of the nucleotide-sugar complexes 224, 224' may be incorporated by polymerases (not specifically illustrated) into respective first oligonucleotides 202 using at least the sequence of respective second oligonucleotides 211, and may be used to characterize second oligonucleotides 211. For example, the presence of the nucleotides of the nucleotide-sugar complexes 224, 224' may be used to determine the presence of respective second oligonucleotides 211, and the identities of the nucleotides of the nucleotide-sugar complexes 224, 224' may be used to determine the identities within respective second oligonucleotides 211 of the nucleotides that are complementary to those nucleotides.

Sugar-lectin couplings may be used to detect the nucleotides of the nucleotide-sugar complexes 224, 224', and in some examples may be used to identify the nucleotides of the nucleotide-sugar complexes. For example, at process 210' illustrated in FIG. 2B, second oligonucleotides 211 may be dehybridized from first oligonucleotides 202. At process 220' illustrated in FIG. 2B, lectins 242 that are multivalent, e.g., divalent, may be coupled to both of the sugars of respective nucleotide-sugar complexes 224, 224', and the nucleotides of those nucleotide-sugar complexes detected using at least detection of the lectins 242. For example, the system illustrated in FIG. 2B may include a composition including substrates 201, nucleotide-sugar complexes 224, 224' coupled to respective substrates (e.g., via respective first oligonucleotides 202'), and multivalent, e.g., divalent, lectins 242, as well as detection circuitry (not specifically illustrated) to detect the nucleotides using at least detection of the respective lectins 242 in a manner similar to that described with reference to FIGS. 1A and 2A. Using at least the selective binding of the different multivalent, e.g., divalent, lectins 242 to the different sugar types of the nucleotide-sugar complexes 224, 224', and the different fluorophores (or other detectable moieties) coupled to those lectins, the different nucleotides of those nucleotide-sugar complexes may be detected using detection circuitry, and may be identified.

It will be appreciated that elements to be detected, i.e. analytes comprising nucleotides, may be coupled to any suitable number and type(s) of sugars. For example, in a manner such as described with reference to FIG. 2B, an element to be detected may be coupled to two sugars that are the same type as one another (illustratively, ddCTP being coupled to two of sugar1). Alternatively, each element may be coupled to two or more sugars that may be different types than one another. For example, FIG. 2C schematically illustrates an example element coupled to multiple sugars. In the nonlimiting example shown in FIG. 2C, element 230 (such as a nucleotide) may be coupled via suitable branched linker 240 to two sugars 224' that are different types than one another. In other examples (not specifically illustrated), element 230 may be coupled via a suitable branched linker to more than two sugars that may be the same type as each other. In still other examples (not specifically illustrated), element 230 may be coupled via a suitable branched linker to more than two sugars that may be different types than each other.

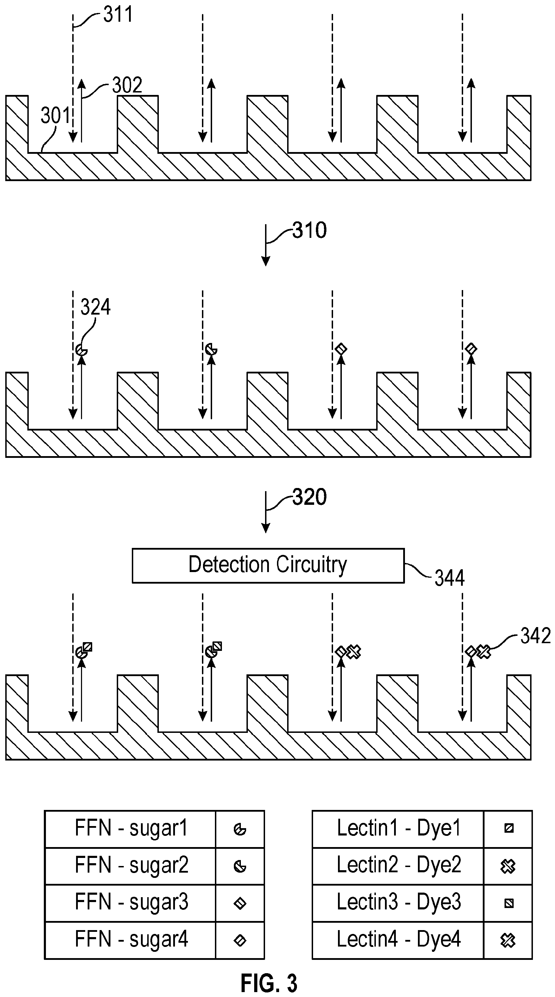

FIG. 3 schematically illustrates another example process flow for detecting an element using sugar-lectin couplings. In the nonlimiting example illustrated in FIG. 3, the element to be detected includes an analyte, such as a nucleotide that may be used in sequencing-by-synthesis process to determine multiple nucleotides in the sequence of an oligonucleotide. Illustratively, a plurality of first oligonucleotides 302 are coupled to respective substrates 301 (a single such first oligonucleotide and a single such substrate being labeled in FIG. 3). Substrates 301 may include respective features on or in a planar substrate as is illustrated in FIG. 3, or may include beads that may be located within respective wells in a flowcell (not specifically illustrated). Each of the first oligonucleotides 302 may, for example, include a sequence that is complementary to a sequence that it is desired to characterize. The sequences of the first oligonucleotides 302 may be the same as one another, or may be different than one another. For example, first oligonucleotides 302 may include primers that are complementary to primers forming part of second oligonucleotides 311. As illustrated in FIG. 3, second oligonucleotides 311 may be hybridized with respective first oligonucleotides 302 and thereby are coupled to respective substrates 301. As such, second oligonucleotides 311 respectively may have sequences with a portion that is complementary to the sequences of the first oligonucleotides 302 to which they are hybridized, and a portion that is to be sequenced.

In a manner similar to that described with reference to FIGS. 1A and 2A, nucleotide-sugar complexes 324 may be coupled to respective substrates 301, and it is desired to detect the presence of the nucleotides or both detect and determine the identity of the nucleotides. Illustratively, at operation 310 a solution may be flowed over the substrates 301 that may include a plurality of element-sugar complexes that include different element types coupled to different sugar types than one another, e.g., a plurality of nucleotide-sugar complexes that include different nucleotide types coupled to different sugar types than one another. For example, nucleotide-sugar complexes 324 may include respective nucleotides coupled to sugars, e.g., via respective linkers. Illustratively, the nucleotide-sugar complexes may include one or more ddNTP-sugar complexes, such as a ddCTP-sugarl complex, a ddGTP-sugar2 complex, a ddATP-sugar3 complex, and a ddTTP-sugar4 (or ddUTP-sugar4) complex, where sugar1, sugar2, sugar3, and sugar4 include different sugar types than one another. The solution also may include polymerases. In a manner similar to that described with reference to FIGS. 1A and 2A, the nucleotides of the nucleotide-sugar complexes 324 may be incorporated by the polymerases (not specifically illustrated) into respective first oligonucleotides 302 using at least the sequence of respective second oligonucleotides 311 so as to extend the first oligonucleotides 302, and may be used to characterize second oligonucleotides 311. For example, the identity of the nucleotides of the nucleotide-sugar complexes 324 may be used to determine the identities within respective second oligonucleotides 311 of the nucleotides that are complementary to those nucleotides.

Sugar-lectin couplings may be used to detect the nucleotides of the nucleotide-sugar complexes 324, and in some examples may be used to identify the nucleotides of the nucleotide-sugar complexes 324. For example, at process 320 illustrated in FIG. 3, lectins 342 may be coupled to the sugars of the nucleotide-sugar complexes 324, and the nucleotides of those nucleotide-sugar complexes 324 detected using at least detection of the lectins 342. For example, the system illustrated in FIG. 3 may include a composition including substrates 301, nucleotide-sugar complexes 324 coupled to respective substrates (e.g., via respective first oligonucleotides 302), and lectins 342, as well as detection circuitry 344 to detect the nucleotides using at least detection of the respective lectins 342. Illustratively, fluorophores may be coupled to respective lectins 342, and detection circuitry 344 may include an optical sensor to detect lectins 342 using at least detection of fluorescence from the fluorophores. For example, lectins 342 may include a Lectin1-Dye1 complex, a Lectin2-Dye2 complex, a Lectin3-Dye3 complex, and a Lectin4-Dye4 complex, where Dye1, Dye2, Dye3, and Dye4 include different fluorophores emitting different wavelengths than one another, and where Lectin1 selectively binds sugar1 and not any of the other sugar types (e.g., does not bind sugar2, sugar3, and sugar4), Lectin2 selectively binds sugar2 and not any of the other sugar types, Lectin3 selectively binds sugar3 and not any of the other sugar types, and Lectin4 selectively binds sugar4 and not any of the other sugar types. That is, Lectin1, Lectin2, Lectin3, and Lectin4 may include different lectin types than one another. Using at least the selective binding of the different lectins 342 to the different sugar types of the nucleotide-sugar complexes 324, and the different fluorophores (or other detectable moieties) coupled to those lectins, the different nucleotides of those nucleotide-sugar complexes 324 may be detected and identified using detection circuitry 344. The lectin and sugar may be cleaved, and operations 310 and 320 repeated any suitable number of times so as to continue extending first oligonucleotides 302 using the sequence of second oligonucleotides 311 while detecting, using sugar-lectin couplings, the identity of the nucleotides added thereby.

From examples such as provided with reference to FIGS. 1A-1B, FIGS. 2A-2B, and FIG. 3, it should be understood that different element-sugar complexes (e.g., different nucleotide-sugar complexes) respectively may be coupled to the same substrate as one another, for example at different times than one another; or may be coupled to different substrates than one another, for example at the same time as one another. Suitable detection circuitry may detect the different elements using detection of different lectins coupled to the sugars of the element-sugar complexes. The different element-sugar complexes may be introduced via a solution that is flowed over structures (such as oligonucleotides) to which the elements, and thus the sugars, and thus the lectins, may be coupled. Such a solution may include any suitable number of different element-sugar complexes, each including an element coupled to a sugar, e.g., two or more, three or more, four or more, or ten or more different element-sugar complexes. In some examples, one or more of the element-sugar complexes may include an additional sugar coupled to the element, e.g., in a manner such as described with reference to FIG. 2B, and the lectin may be divalent and may selectively bind both of the sugars coupled to the element.

Any suitable sugars may be included in the present element-sugar complexes, and any suitable lectins may selectively bind such sugars. For example, one or more of the sugars may include an alkyl sugar, e.g., a sugars that includes an alkyl group via which the sugar may be coupled to an element, such as a nucleotide. One or more of the sugars may include a monosaccharide. One or more of the sugars may include a disaccharide. One or more of the sugars may include a polysaccharide. In some examples, the lectin includes Concanavalin A (Con A) and the sugar of the element-sugar complex includes mannose (Man) or glucose (Glc). In other examples, the lectin includes wheat germ agglutinin (WGA) and the sugar of the element-sugar complex includes N-acetyl-glucosamine (GlcNAc) or N-acetyl-neuraminic acid (Neu5Ac). In other examples, the lectin includes soybean agglutinin (SBA) and the sugar of the element-sugar complex includes galactose (Gal) or N-acetyl-galactosamine (GalNAc). In other examples, the lectin includes *Dolichos bifloris* (DBA) and the sugar of the element-sugar complex includes GalNAcα3GalNAc or GalNAc. In other examples, the lectin includes ricin and the sugar of the element-sugar complex includes galactose. In other examples, the lectin includes peanut agglutinin (PNA) and the sugar of the element-sugar complex includes galactose or Galβ3GalNAcα (T-antigen). In other examples, the lectin includes *Pisum sativum* (PSA) and the sugar of the element-sugar complex includes mannose or glucose. In other examples, the lectin includes *Lens culinaris* (LCA) and the sugar of the element-sugar complex includes mannose or glucose. In other examples, the lectin includes *Galanthus nivalus* (GNA) and the sugar of the element-sugar complex includes mannose. In other examples, the lectin includes *Solanum tuberosum* (STA) and the sugar of the element-sugar complex includes (GlcNAc)ₙ. In other examples, the lectin includes Asialoglycoprotein receptor (ASGPR) H1 and the sugar of the element-sugar complex includes galactose. In other examples, the lectin includes Galectin-3 and the sugar of the element-sugar complex includes galactose. In other examples, the lectin includes Sialoadhesin and the sugar of the element-sugar complex includes Neu5Ac. In other examples, the lectin includes Cation-dependent mannose-6-phosphate receptor (CD-MPR) and the sugar of the element-sugar complex includes Man6P. In other examples, the lectin includes C-reactive protein (CRP) and the sugar of the element-sugar complex includes galactose, Gal6P, or galacturonic acid. Other examples of suitable lectins are described elsewhere herein, and the sugars of the sugar-element complexes may include any suitable sugars that such lectins selectively bind.

Illustratively, the nucleotides of the nucleotide-sugar complexes may be or include ddNTPs, such as ddUTP, ddTTP, ddATP, ddCTP, or ddGTP. The nucleotides may be coupled to the sugars via respective linkers, such as PEG (e.g., PEG₁₋₄₈), alkyl, or peptide. In one nonlimiting example, a nucleotide-sugar complex is ddUTP-PEG7-mannose having the structure shown below:

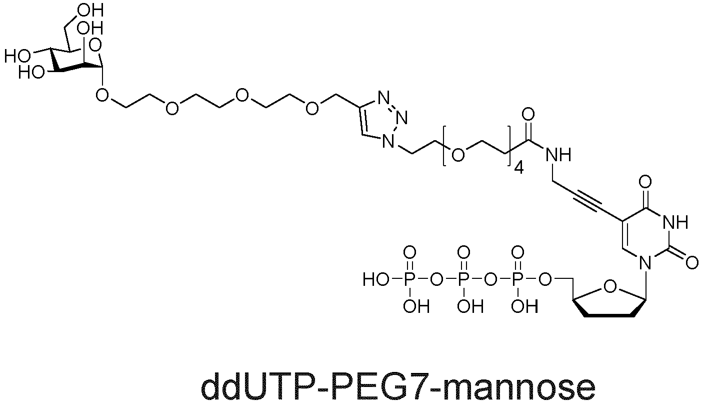

In another nonlimiting example, a nucleotide-sugar complex is ddATP-PEG7-mannose having the structure shown below:

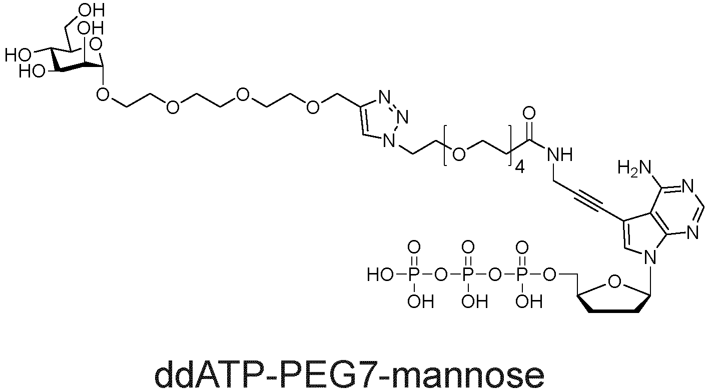

In another nonlimiting example, a nucleotide-sugar complex is ddCTP-PEG7-β-GlcNAc having the structure shown below:

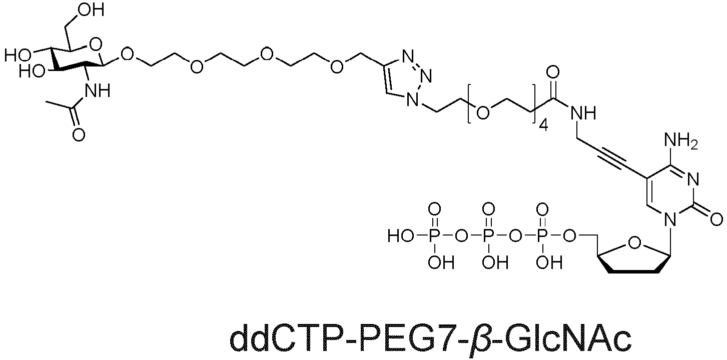

In another nonlimiting example, a nucleotide-sugar complex is ddGTP-PEG7-β-GlcNAc having the structure shown below:

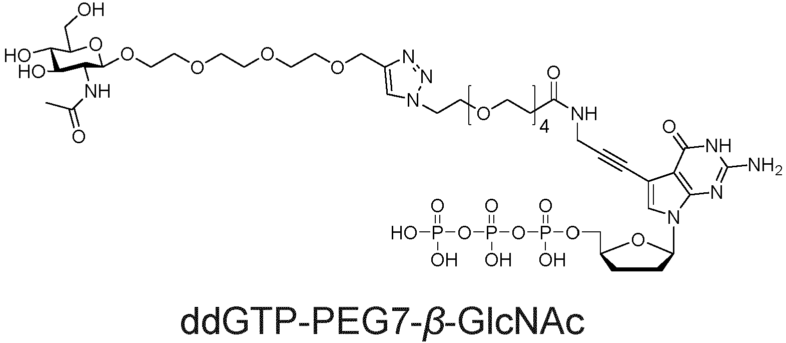

An example reaction scheme for preparing nucleotide-sugar complexes such as, but not limited to, ddUTP-PEG7-mannose, ddATP-PEG7-mannose, ddCTP-PEG7-β-GlcNAc, and ddGTP-PEG7-β-GlcNAc is provided below in Example 3.

In some examples, different element types are coupled to different ones of the different sugar types such as listed above, and lectins selective of such sugars such as listed above are coupled to such sugar types and used to detect the elements that are coupled to those sugar types.

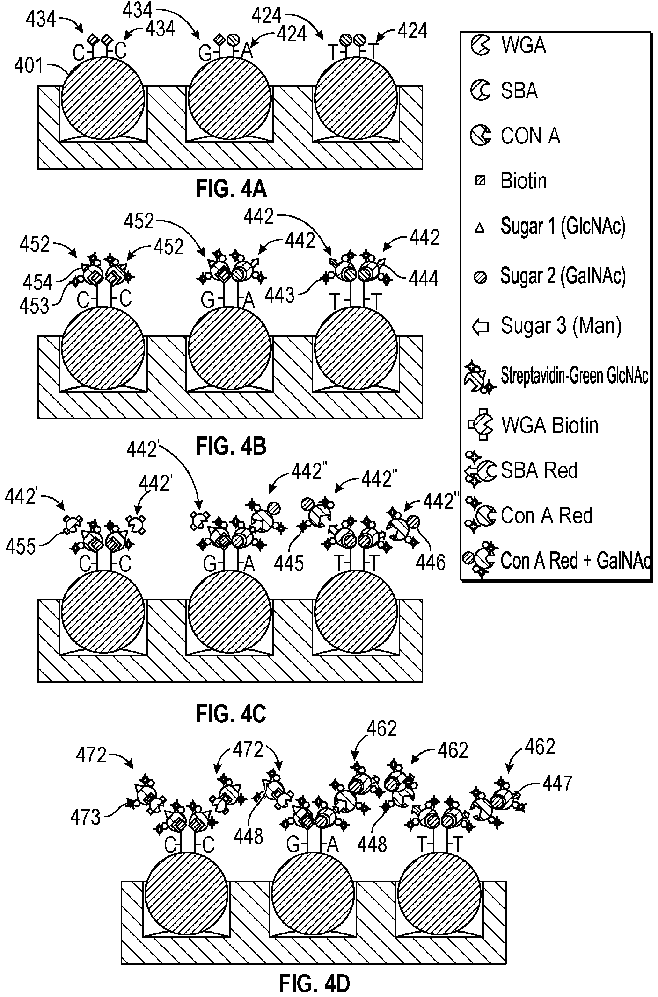

In some examples, while one or more different element types may coupled to different sugar types such as listed above, and lectins selective of such sugars such as listed above are coupled to such sugar types and used to detect the elements that are coupled to those sugar types, other element types may be coupled to nonsugar moieties that are selectively bound by nonlectin proteins that are selective of those moieties. For example, FIGS. 4A-4D schematically illustrate an example process flow for detecting elements using a combination of sugar-lectin couplings and one or more other types of couplings. In FIG. 4A, first oligonucleotides are coupled to respective substrates 401. In a manner similar to that described with reference to FIGS. 1A and 2A, a plurality of nucleotide complexes are coupled to the substrates via respective first oligonucleotides using the sequence of second oligonucleotides and the second oligonucleotides then removed (first and second oligonucleotides not expressly illustrated). In the example shown in FIG. 4A, the nucleotide complexes include one or more nucleotide-sugar complexes 424 such as described elsewhere herein, as well as one or more nucleotide-nonsugar moiety complexes 434. In the nonlimiting example shown in FIG. 4A, the nucleotide-sugar complexes 424 include GalNAc, but it should be appreciated that any suitable sugar type may be used. The nucleotide-nonsugar moiety complex(es) may include nucleotides that are coupled to a moiety for which a nonlectin protein is selective. In the nonlimiting example shown in FIG. 4A, the nonsugar moiety of complexes 434 may include biotin, but it should be appreciated that any suitable nonsugar moiety may be used.

FIG. 4B illustrates selective binding of lectins 442 to the sugars of complexes 424, and the selective binding of nonlectin proteins 452 to the moieties of complexes 434. In the nonlimiting example shown in FIG. 4B, lectins 442 include SBA, but it should be appreciated that any suitable lectin types(s) may be used that are selective for the sugar type(s) of complexes 424. In the nonlimiting example shown in FIG. 4B, nonlectin proteins 452 include streptavidin, but it should be appreciated that any suitable nonlectin protein type(s) may be used that are selective for the nonsugar moiety type(s) of complexes 434. Each of lectins 442 may include a fluorophore 443, e.g., may include a plurality of fluorophores 443. Each of nonlectin proteins 452 may include a fluorophore 453, e.g., may include a plurality of fluorophores 453, which may be of a different type than fluorophores 443 so as to permit lectins 442 to be optically distinguished from nonlectin proteins 452 using suitable detection circuitry (not specifically illustrated). For example, fluorophores 443 may be red, while fluorophores 453 may be green, although any suitable colors and types of fluorophores may be used.

So as to further increase the number of fluorophores that may be coupled to each of the complexes 424, 434, and thus increase detectability and identification of the nucleotides of such complexes, additional lectins or nonlectin proteins may be coupled to the lectins or nonlectin proteins that are already coupled to the complexes. Additional fluorophores may be coupled to the additional lectins or nonlectin proteins. For example, as illustrated in FIG. 4B, lectins 442 may include sugars 444, and nonlectin proteins 452 may include sugars 454. In the nonlimiting example shown in FIG. 4B, sugars 444 include mannose (Man) and sugars 454 include GlcNAc, but it should be appreciated that any suitable sugar types may be used. FIG. 4C illustrates the coupling of second lectins 442' to sugars 454, and the coupling of third lectins 442" to sugars 444. In the nonlimiting example illustrated in FIG. 4C, second lectins 442' include WGA and third lectins 442" include Con A, but it should be appreciated that any suitable lectin types may be used that selectively bind sugars that are included in lectins 442 or nonlectin proteins 452. The second and third lectins 442', 442" may include fluorophores, or may include additional moieties via which fluorophores may be coupled thereto. For example, second lectins 442' may include nonsugar moieties 455 to which additional fluorophores may be coupled via additional nonlectin proteins in a manner such as described below with reference to FIG. 4D. As another example, third lectins 442" may include fluorophores 445, which may be the same type as, or different than, one or both of fluorophores 443 or 453. Additionally, or alternatively, third lectins 442" may include sugars 446 to which additional fluorophores may be coupled via additional lectins in a manner such as described below with reference to FIG. 4D. In the nonlimiting example illustrated in FIG. 4C, nonsugar moieties 455 include biotin and sugars 446 include GalNAc, but it should be appreciated that any suitable nonsugar moieity types and sugar types may be used.

FIG. 4D illustrates selective binding of fourth lectins 462 to sugars 446, and the selective binding of second nonlectin proteins 472 to nonsugar moieties 455. In the nonlimiting example shown in FIG. 4D, fourth lectins 462 include SBA, but it should be appreciated that any suitable lectin type(s) may be used that are selective for sugars 446. In the nonlimiting example shown in FIG. 4D, nonlectin proteins 472 include streptavidin, but it should be appreciated that any suitable nonlectin protein type(s) may be used that are selective for the nonsugar moieties 455. Each of lectins 462 may include a fluorophore 448, e.g., may include a plurality of fluorophores 448. Each of nonlectin proteins 472 may include a fluorophore 473, e.g., may include a plurality of fluorophores 473, which may be of a different type than fluorophores 473 so as to permit lectins 462 to be optically distinguished from nonlectin proteins 472 using suitable detection circuitry (not specifically illustrated). For example, fluorophores 448 may be red, while fluorophores 473 may be green, although any suitable colors and types of fluorophores may be used. In a manner similar to that described with reference to FIGS. 4B-4D, lectins 462 and nonlectin proteins 472 may include additional sugars or nonsugar moieties (e.g., sugars 447 such as mannose or sugars 448 such as GlcNAc) via which still further lectins may be coupled so as to detect complexes 424, 434.

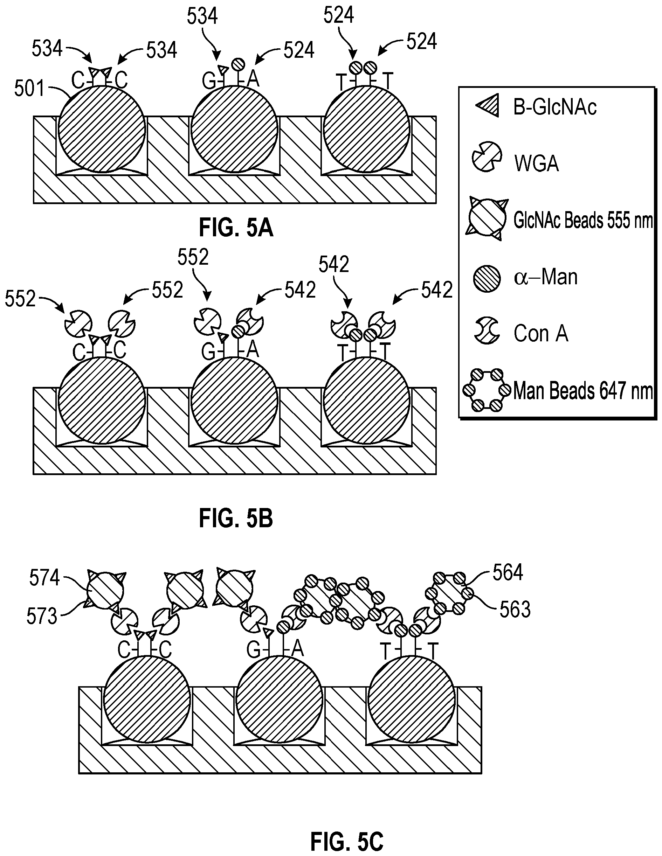

Although FIGS. 4A-4D illustrate an example in which a mixture of lectins and nonlectin proteins is used to detect analytes such as nucleotides, will be appreciated that in other examples a mixture of lectins that excludes nonlectin proteins alternatively may be used. Illustratively, FIGS. 5A-5C schematically illustrate another example process flow for detecting elements using sugar-lectin couplings. In FIG. 5A, first oligonucleotides are coupled to respective substrates 501. In a manner similar to that described with reference to FIGS. 1A and 2A, a plurality of nucleotide complexes are coupled to the substrates via respective first oligonucleotides using the sequence of second oligonucleotides and the second oligonucleotides then removed (first and second oligonucleotides not expressly illustrated). In the example shown in FIG. 5A, the nucleotide complexes include first nucleotide-sugar complexes 524 such as described elsewhere herein, as well as one or more second nucleotide-sugar complexes 534. In the nonlimiting example shown in FIG. 5A, the first nucleotide-sugar complexes 524 include α-mannose and the second nucleotide-sugar complexes 534 include B-GlcNAc, but it should be appreciated that any suitable sugar types may be used.

FIG. 5B illustrates selective binding of lectins 542 to the sugars of complexes 524, and the selective binding of lectins 552 to the sugars of complexes 534. In the nonlimiting example shown in FIG. 5B, lectins 542 include Con A and lectins 552 include WGA, but it should be appreciated that any suitable lectin types may be used that respectively are selective for the sugars of complexes 524, 524. Fluorophores may be coupled to lectins 542, 552 before the lectins are coupled to the sugars, or may be coupled to lectins 542, 552 after the lectins are coupled to the sugars. For example, each of lectins 542 and 552 may include a fluorophore, e.g., may include a plurality of fluorophores (fluorophore(s) not specifically illustrated), which may be of different types than one another so as to permit lectins 542 to be optically distinguished from lectins 552 using suitable detection circuitry (circuitry not specifically illustrated).

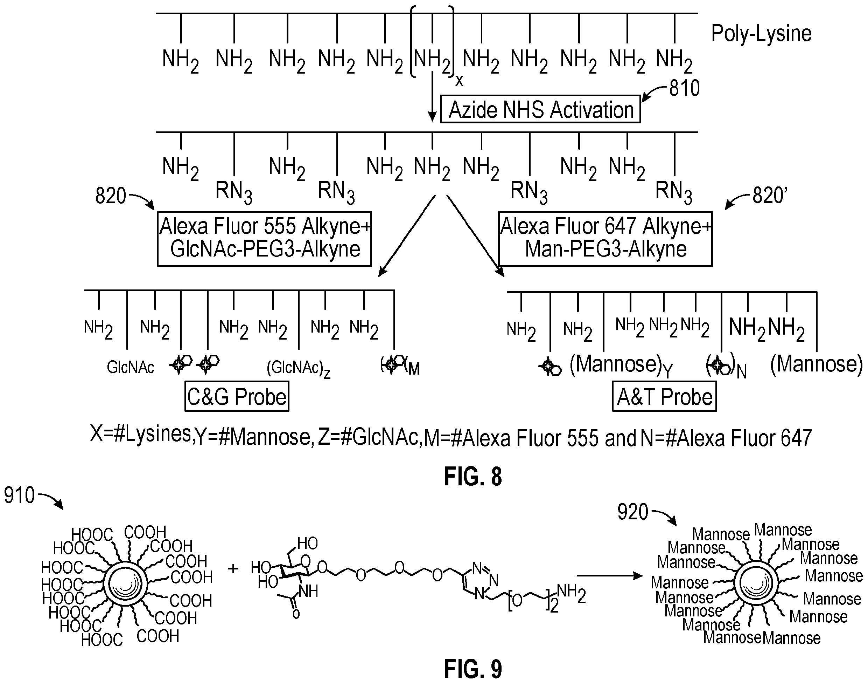

Alternatively, fluorophore(s) may be coupled to the lectins in a subsequent step. For example, as illustrated in FIG. 5B, lectins 542 and 552 may be divalent. As such, an additional sugar may be coupled to each of lectins 542, 552 besides the sugar to which the lectins are already coupled, e.g., in a manner such as illustrated in FIG. 5C. The additional sugar may be coupled to any suitable structure(s) via which the lectins are detectable. In the nonlimiting example illustrated in FIG. 5C, additional sugars 563 are coupled to lectins 542, and also are coupled to polymers via which the lectins may be detected, e.g., to fluorescent beads 564 that emit a first wavelength; and additional sugars 573 are coupled to lectins 552, and also are coupled to polymers via which the lectins may be detected, e.g., to fluorescent beads 574 that emit a second, different wavelength. Illustratively, additional sugars 563 may include mannose and additional sugars 573 may include GlCNac, but it should be appreciated that any suitable sugars may be used for which lectins 542, 552 respectively are selective. Fluorescent beads 564 may be red (e.g., may fluoresce at 647 nm) and fluorescent beads 574 may be green (e.g., may fluoresce at 555 nm), but it should be appreciated that any suitable fluorescent beads may be used. Additionally, it should be appreciated that still further lectins, and additional fluorescent beads, may be coupled to additional sugars 563, 573 in a manner similar to that described with reference to FIGS. 4C-4D. Fluorescent beads that include sugars, such as described with reference to FIG. 5C, may be prepared using any suitable sequence of steps, for example such as described with reference to FIG. 9.

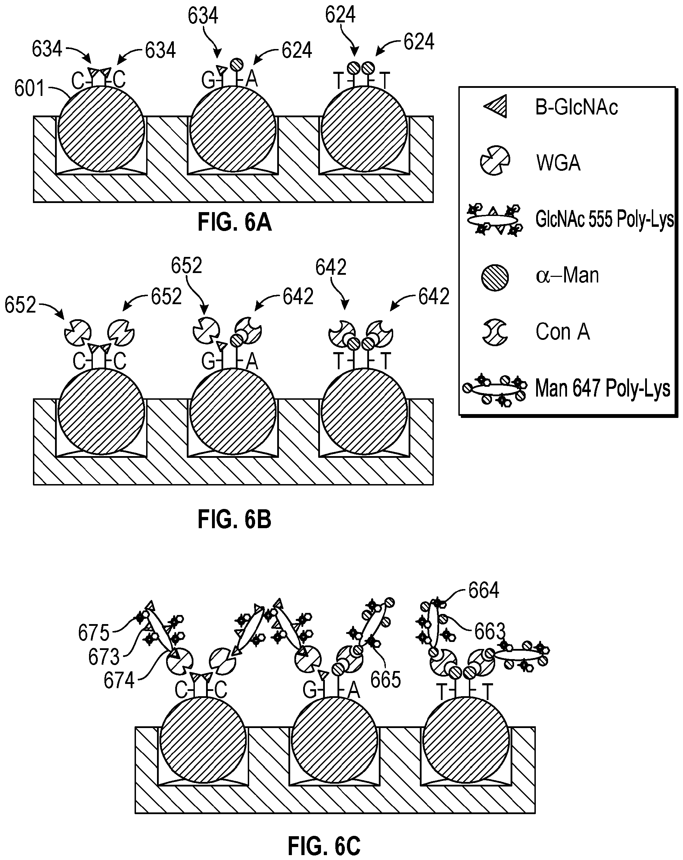

FIGS. 6A-6C schematically illustrate another example process flow for detecting elements using sugar-lectin couplings. In FIG. 6A, first oligonucleotides are coupled to respective substrates 601. In a manner similar to that described with reference to FIGS. 1A and 2A, a plurality of nucleotide complexes are coupled to the substrates via respective first oligonucleotides using the sequence of second oligonucleotides and the second oligonucleotides then removed (first and second oligonucleotides not expressly illustrated). In the example shown in FIG. 6A, the nucleotide complexes include first nucleotide-sugar complexes 624 such as described elsewhere herein, as well as one or more second nucleotide-sugar complexes 634. In the nonlimiting example shown in FIG. 6A, the first nucleotide-sugar complexes 624 include α-mannose and the second nucleotide-sugar complexes 634 include B-GlcNAc, but it should be appreciated that any suitable sugar types may be used.

FIG. 6B illustrates selective binding of lectins 642 to the sugars of complexes 624, and the selective binding of lectins 652 to the sugars of complexes 634. In the nonlimiting example shown in FIG. 6B, lectins 642 include Con A and lectins 652 include WGA, but it should be appreciated that any suitable lectin types may be used that respectively are selective for the sugars of complexes 624, 624. Fluorophores may be coupled to lectins 642, 652 before the lectins are coupled to the sugars, or may be coupled to lectins 642, 652 after the lectins are coupled to the sugars. For example, each of lectins 642 and 652 may include a fluorophore, e.g., may include a plurality of fluorophores (fluorophore(s) not specifically illustrated), which may be of different types than one another so as to permit lectins 642 to be optically distinguished from lectins 652 using suitable detection circuitry (circuitry not specifically illustrated).

Alternatively, fluorophore(s) may be added to the lectins in a subsequent step. For example, as illustrated in FIG. 6B, lectins 642 and 652 may be divalent. As such, an additional sugar may be coupled to each of lectins 642, 652 besides the sugar to which the lectins are already coupled, e.g., in a manner such as illustrated in FIG. 6C. The additional sugar may be coupled to any suitable structure(s) via which the lectins are detectable. In the nonlimiting example illustrated in FIG. 6C, additional sugars 663 are coupled to lectins 642, and also are coupled to polymers via which the lectins may be detected, e.g., to polypeptides 664 including fluorophores 665 that emit a first wavelength; and additional sugars 673 are coupled to lectins 652, and also are coupled to polymers via which the lectins may be detected, e.g., to polypeptides 674 including fluorophores 675 that emit a second, different wavelength. Polypeptides 664, 674 may include polypeptide rings, e.g., rings including polylysine or other suitable peptide or combination of peptides. The rings may provide localized areas of fluorescence and sugar molecules. Because lysine includes amino side chains, rings including polylysine readily may be functionalized (e.g., so as to include fluorophores and sugar molecules) via amide coupling reactions with such lysine side chains. Illustratively, additional sugars 663 may include mannose and additional sugars 673 may include GlCNac, but it should be appreciated that any suitable sugars may be used for which lectins 642, 652 respectively are selective. Fluorophores 665may be red (e.g., may fluoresce at 647 nm) and fluorophores 675may be green (e.g., may fluoresce at 555 nm), but it should be appreciated that any suitable fluorophores may be used. Additionally, it should be appreciated that still further lectins, and additional polypeptides, may be coupled to additional sugars 663 in a manner similar to that described with reference to FIGS. 4C-4D. Polypeptides that include sugars and fluorophores, such as described with reference to FIG. 6C, may be prepared using any suitable sequence of steps, for example such as described with reference to FIG. 8.

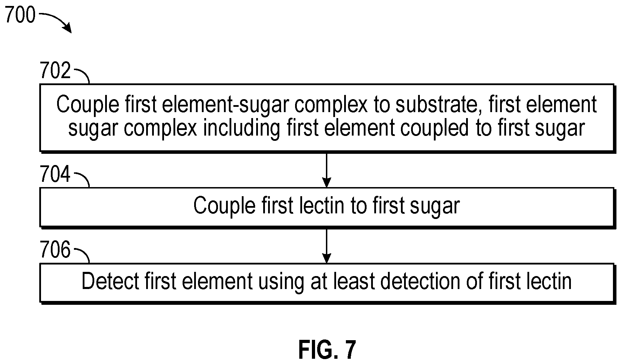

Systems and compositions such as described herein may be adapted for use in any suitable method for detecting an element. Illustratively, FIG. 7 schematically illustrates another example process flow for detecting an element using a sugar-lectin coupling. Process flow 700 illustrated in FIG. 7 may include coupling a first element-sugar complex to a first substrate, the first element-sugar complex including a first element coupled to a first sugar (operation 702). For example, nucleotide-sugar complex 121 may be coupled to substrate 101 via first oligonucleotide 102' in a manner such as described with reference to FIG. 1A. Or, for example, nucleotide-sugar complex 121' may be coupled to substrate 101' via first oligonucleotide 102 in a manner such as described with reference to FIG. 1B. Or, for example, a first one of nucleotide-sugar complexes 224 may be coupled to substrate 201 via first oligonucleotide 202 in a manner such as described with reference to FIG. 2A. Or, for example, a first one of nucleotide-sugar complexes 224' may be coupled to substrate 201' via first oligonucleotide 202' in a manner such as described with reference to FIG. 2B. Or, for example, a first one of nucleotide-sugar complexes 324 may be coupled to substrate 301 via a first oligonucleotide 302 in a manner such as described with reference to FIG. 3. Or, for example, a first one of nucleotide-sugar complexes 424 may be coupled to substrate 401 in a manner such as described with reference to FIG. 4A. Or, for example, a first one of nucleotide-sugar complexes 524 or 534 may be coupled to substrate 501 in a manner such as described with reference to FIG. 5A. Or, for example, a first one of nucleotide-sugar complexes 624, 634 may be coupled to substrate 601 in a manner such as described with reference to FIG. 6A. In some examples, the first substrate includes a bead, e.g., as described with reference to FIGS. 1A-1B, FIGS. 2A-2B, FIGS. 4A-4D.

Process flow 700 illustrated in FIG. 7 may include coupling a first lectin to the first sugar (operation 704). For example, lectin 142 may be coupled to nucleotide-sugar complex 121 in a manner such as described with reference to FIG. 1A. Or, for example, lectin 142' may be coupled to nucleotide-sugar complex 121' in a manner such as described with reference to FIG. 1B. Or, for example, a first one of lectins 242 may be coupled to the first one of nucleotide-sugar complexes 224 in a manner such as described with reference to FIG. 2A. Or, for example, a first one of lectins 242' may be coupled to the first one of nucleotide-sugar complexes 224' in a manner such as described with reference to FIG. 2B. Or, for example, a first one of lectins 342 may be coupled to the first one of the nucleotide-sugar complexes 324 in a manner such as described with reference to FIG. 3. Or, for example, a first one of lectins 442 may be coupled to the first one of the nucleotide-sugar complexes 424 in a manner such as described with reference to FIG. 4B. Or, for example, a first one of lectins 542 or 552 may be coupled to the first one of the nucleotide-sugar complexes 524 or 534 in a manner such as described with reference to FIG. 5B. Or, for example, a first one of lectins 642 or 652 may be coupled to the first one of the nucleotide-sugar complexes 624, 634 in a manner such as described with reference to FIG. 6B.

Process flow 700 illustrated in FIG. 7 may include detecting the first element using at least detection of the first lectin (operation 706). For example, lectin 142 may be detected using one or more fluorophore(s) 143 in a manner such as described with reference to FIG. 1A. Or, for example, lectin 142' may be detected using one or more fluorophore(s) 143' in a manner such as described with reference to FIG. 1B. Or, for example, the first one of lectins 242 may be detected using one or more fluorophore(s), such as Dye1, Dye2, Dye3, or Dye4 in a manner such as described with reference to FIG. 2A. Or, for example, the first one of lectins 242' may be detected using one or more fluorophore(s), such as Dye1, Dye2, Dye3, or Dye4 in a manner such as described with reference to FIG. 2B. Or, for example, the first one of lectins 242' may be detected using one or more fluorophore(s), such as Dye1, Dye2, Dye3, or Dye4 in a manner such as described with reference to FIG. 3. Or, for example, the first one of lectins 442 may be detected using one or more fluorophore(s) 443 in a manner such as described with reference to FIG. 4B. Or, for example, the first one of lectins 542 or 552 may be detected using one or more fluorescent beads 564, 574 in a manner such as described with reference to FIG. 5C. Or, for example, the first one of lectins 642 or 652 may be detected using one or more fluorophores 665, 675 in a manner such as described with reference to FIG. 6C.

In some examples of process flow 700 described with reference to FIG. 7, the first element includes an analyte. Examples of different analytes are provided elsewhere herein. The analyte includes a first nucleotide, e.g., such as described with reference to FIGS. 1A-1B, FIGS. 2A-2B, FIG. 3, FIGS. 4A-4D, FIGS. 5A-5C, or FIGS. 6A-6C. Coupling the first element-sugar complex to the first substrate includes incorporating the first nucleotide into a first oligonucleotide coupled to the first substrate, e.g., in a manner such as described with reference to FIGS. 1A-1B, FIGS. 2A-2B, FIG. 3, FIGS. 4A-4D, FIGS. 5A-5C, or FIGS. 6A-6C. The first oligonucleotide is hybridized to a second oligonucleotide, e.g., in a manner such as described with reference to FIGS. 1A-1B, FIGS. 2A-2B, FIG. 3, FIGS. 4A-4D, FIGS. 5A-5C, or FIGS. 6A-6C. Incorporating the first nucleotide into the first oligonucleotide includes extending the first oligonucleotide using at least a sequence of the second oligonucleotide, e.g., in a manner such as described with reference to FIGS. 1A-1B, FIGS. 2A-2B, FIG. 3, FIGS. 4A-4D, FIGS. 5A-5C, or FIGS. 6A-6C.

In some examples, coupling the first element-sugar complex to the first substrate may include flowing a solution over the substrate. The solution may include the first element-sugar complex, and a second element-sugar complex including a second element coupled to a second sugar, e.g., in a manner such as described with reference to FIGS. 2A-2B and FIG. 3, and which also may be implemented in the examples described with reference to FIGS. 1A-1B, FIGS. 4A-4D, FIGS. 5A-5C, or FIGS. 6A-6C. The first lectin selectively may binds the first sugar and does not bind the second sugar. The first and second elements may include different nucleotide types than one another. The first and second sugars may include different sugar types than one another. Process flow 700 may include coupling the second element-sugar complex to the first substrate or to a second substrate. For example, the second element-sugar complex may be coupled to the same (first) substrate as is the first element-sugar complex, e.g.., in a manner such as described with reference to second nucleotide-sugar complex 164' illustrated in FIG. 1B, or a second one of the nucleotide-sugar complexes 424 that is coupled to the same substrate 401 as the first one of the nucleotide-sugar complexes 424 in a manner such as described with reference to FIG. 4A, or a second one of the nucleotide-sugar complexes 524 that is coupled to the same substrate 501 as the first one of the nucleotide-sugar complexes 524 in a manner such as described with reference to FIG. 5A, or a second one of the nucleotide-sugar complexes 624 that is coupled to the same substrate 601 as the first one of the nucleotide-sugar complexes 624 in a manner such as described with reference to FIG. 6B. Or, for example, the second-element sugar complex may be coupled to a different (second) substrate than the first element-sugar complex, e.g., in a manner such as described with reference to a second one of the nucleotide-sugar complexes 224 that is coupled to a different substrate 201 than the first one of the nucleotide-sugar complexes 224 in a manner such as described with reference to FIG. 2A, or a second one of the nucleotide-sugar complexes 224' that is coupled to a different substrate 201' than the first one of the nucleotide-sugar complexes 224' in a manner such as described with reference to FIG. 2B, or a second one of the nucleotide-sugar complexes 324 that is coupled to a different substrate 301 than the first one of the nucleotide-sugar complexes 324 in a manner such as described with reference to FIG. 4A, or a second one of the nucleotide-sugar complexes 424 that is coupled to a different substrate 401 than the first one of the nucleotide-sugar complexes 424 in a manner such as described with reference to FIG. 4A, or a second one of the nucleotide-sugar complexes 524 that is coupled to a different substrate 501 than the first one of the nucleotide-sugar complexes 524 in a manner such as described with reference to FIG. 5A, or a second one of the nucleotide-sugar complexes 624 that is coupled to a different substrate 601 than the first one of the nucleotide-sugar complexes 624 in a manner such as described with reference to FIG. 6A.

Process flow 700 further may include coupling a second lectin to the second sugar, and detecting the second element using at least detection of the second lectin. For example, second lectin 172' may be coupled to second sugar 162' in a manner such as described with reference to FIG. 1B, or a second one of lectins 242 may be coupled to the sugar of the second one of the nucleotide-sugar complexes 224 in a manner such as described with reference to FIG. 2A, or a second one of lectins 242' may be coupled to the sugar of the second one of the nucleotide-sugar complexes 224' in a manner such as described with reference to FIG. 2B, or a second one of lectins 342 may be coupled to the sugar of the second one of the nucleotide-sugar complexes 324 in a manner such as described with reference to FIG. 3, or a second one of lectins 442 may be coupled to the sugar of the second one of the nucleotide-sugar complexes 424 in a manner such as described with reference to FIG. 4B, or a second one of lectins 542, 552 may be coupled to the sugar of the second one of the nucleotide-sugar complexes 524, 534 in a manner such as described with reference to FIG. 5B, or a second one of lectins 642, 652 may be coupled to the sugar of the second one of the nucleotide-sugar complexes 624, 634 in a manner such as described with reference to FIG. 6B.

The solution further may include a third element-sugar complex including a third element coupled to a third sugar; and a fourth element-sugar complex including a fourth element coupled to a fourth sugar. Process flow 700 further may include coupling the third element-sugar complex to the first substrate, to the second substrate, or to a third substrate; coupling a third lectin to the third sugar; detecting the third element using at least detection of the third lectin; coupling the fourth element-sugar complex to the first substrate, to the second substrate, to the third substrate, or to a fourth substrate; coupling a fourth lectin to the fourth sugar; and detecting the fourth element using at least detection of the fourth lectin, e.g., in a manner such as described with reference to FIGS. 2A-2B, FIG. 3, FIGS. 4A-4D, FIGS. 5A-5C, or FIGS. 6A-6C.

In some examples, the first element sugar-complex further includes a fifth sugar coupled to the element. For example, in a manner such as described with reference to FIG. 2B, nucleotide-sugar complexes 224' may include additional sugars coupled to the nucleotide. Process flow 700 may include coupling the first lectin to the fifth sugar. For example, in a manner such as described with reference to FIG. 2B, one or more of the lectins may be divalent, and thus may be coupled to both sugars of the respective nucleotide-sugar complex. It will be appreciated that divalent lectins may be used in any other examples provided herein, e.g., such as described with reference to FIGS. 1A-1B, FIG. 2A, FIG. 3, FIGS. 4A-4D, FIGS. 5A-5C, or FIGS. 6A-6C.

In some examples, the process flow may include coupling a first fluorophore to the first lectin, wherein detection of the first lectin includes detecting fluorescence from the first fluorophore. Illustratively, lectin 142, lectin 142', lectin 242, lectin 242', lectin 342, lectin 442, lectin 542, lectin 552, lectin 642, or lectin 642' may include, or may be coupled to, a first fluorophore. The first fluorophore may include a first plurality of fluorophores. Coupling the first fluorophore to the first lectin may include coupling a polymer to the first lectin, wherein the polymer includes the first fluorophore and a sixth sugar, wherein the first lectin couples to the sixth sugar. For example, in a manner such as described with reference to FIG. 5C, the polymer may include fluorescent bead 564 that includes sugar 563 to which lectin 542 couples, or fluorescent bead 574 that includes sugar 573 to which lectin 552 couples. Or, for example, in a manner such as described with reference to FIG. 6C, the polymer may include polypeptide 664 that includes fluorophore 665 as well as sugar 663 to which lectin 642 couples, or polypeptide 674 that includes fluorophore 675 as well as sugar 673 to which lectin 652 couples.

The first fluorophore may be coupled to the first lectin before coupling the first lectin to the first sugar, e.g., in a manner such as described with reference to FIGS. 1A-1B, 2A-2B, or FIG. 3. Alternatively, the first fluorophore may be coupled to the first lectin after coupling the first lectin to the first sugar, e.g., in a manner such as described with reference to FIGS. 4A-4D, 5A-5C, or 6A-6C.

In some examples, a seventh sugar may be coupled to the first lectin, a second lectin may be coupled to the seventh sugar, and detection of the first lectin includes detection of the second lectin. For example, in a manner such as described with reference to FIGS. 4B-4C, the first one of the lectins 442 may include a sugar 444 to which a second lectin 442" may be coupled, and second lectin 442" may be detected, e.g., using fluorescence. Illustratively, a second fluorophore 445 may be coupled to the second lectin 442", and wherein detection of the first lectin may include detecting fluorescence from the second fluorophore. The first fluorophore may be a different type of fluorophore than the second fluorophore, or may be the same type of fluorophore as the second fluorophore. In one specific, nonlimiting example such as described with reference to FIGS. 4B-4C, first lectin 442 includes Concanavalin A (Con A), the seventh sugar 444 includes N-acetyl-galactosamine (GalNAc), and the second lectin 442" includes soybean agglutinin (SBA).

In some examples, the first sugar includes an alkyl sugar, e.g., such as described in Example 3. The first sugar may include a monosaccharide, or may include a disaccharide.

In various example implementations of process flow 700, the first lectin includes Concanavalin A (Con A) and the first sugar includes mannose (Man) or glucose (Glc); the first lectin includes wheat germ agglutinin (WGA) and the first sugar includes N-acetyl-glucosamine (GlcNAc) or N-acetyl-neuraminic acid (Neu5Ac); the first lectin includes soybean agglutinin (SBA) and the first sugar includes galactose (Gal) or N-acetyl-galactosamine (GalNAc); the first lectin includes *Dolichos bifloris* (DBA) and the first sugar includes GalNAcα3GalNAc or GalNAc; the first lectin includes Ricin and the first sugar includes galactose; the first lectin includes peanut agglutinin (PNA) and the first sugar includes galactose or Galβ3GalNAcα (T-antigen); the first lectin includes *Pisum sativum* (PSA) and the first sugar includes mannose or glucose; the first lectin includes *Lens culinaris* (LCA) and the first sugar includes mannose or glucose; the first lectin includes *Galanthus nivalus* (GNA) and the first sugar includes mannose; the first lectin includes *Solanum tuberosum* (STA) and the first sugar includes (GlcNAc)ₙ; the first lectin includes Asialoglycoprotein receptor (ASGPR) H1 and the first sugar includes galactose; the first lectin includes Galectin-3 and the first sugar includes galactose; the first lectin includes Sialoadhesin and the first sugar includes Neu5Ac; the first lectin includes Cation-dependent mannose-6-phosphate receptor (CD-MPR) and the first sugar includes Man6P; or the first lectin includes C-reactive protein (CRP) and the first sugar includes galactose, Gal6P, or galacturonic acid.

### ADDITIONAL EXAMPLES

Additional examples are disclosed in further detail in the following examples, which are not in any way intended to limit the scope of the claims.

### Example 1. Coupling sugars and fluorophores to polypeptides

FIG. 8 schematically illustrates an example process flow for coupling sugars and fluorophores to a polypeptide. At operation 810, a number (X) of lysines within a polylysine strand are functionalized so as to include azido (RN₃) groups, e.g., using Azide succinimidyl ester (A10280 from ThermoFisher Scientific), where R is a linker such as PEG, alkyl, or peptide. At operation 820, a first portion of the azido-functionalized polylysine resulting from operation 810 is mixed in solution with the fluorophore Alexa Fluor 555 Alkyne (A20013 from ThermoFisher Scientific) and the alkyl sugar β-GlcNAc-PEG3-Alkyne (SMB00414 from Sigma) to obtain polylysine including a number (*Z*) of the sugar GlcNAc and a number (*M*) of the fluorophore Alexa Fluor 555. At operation 820', a second portion of the azido-functionalized polylysine resulting from operation 810 is mixed in solution with the fluorophore Alexa Fluor 647 Alkyne (A10278 from ThermoFisher Scientific) and the alkyl sugar α-Man-PEG3-Alkyne (SMB00415 from Sigma) to obtain polylysine including a number (*Y*) of the sugar mannose and a number (*N*) of the fluorophore Alexa Fluor 647. In some examples, Xis between about 50 and about 200, e.g., between about 100 and about 150. In some examples, *Y* is between about 10 and about 50, e.g., between about 20 and about 40. In some examples, *Z* is between about 10 and about 50, e.g., between about 20 and about 40. In some examples, *M* is between about 5 and about 50, e.g., between about 15 and about 40. In some examples, *N* is between about 5 and about 50, e.g., between about 15 and about 40. The polypeptide resulting from operation 820 is used to fluorescently label the lectin Con A in the manner illustrated in FIG. 6C. The polypeptide resulting from operation 820' is used to fluorescently label the lectin WGA in the manner illustrated in FIG. 6C. From this example it may be understood that sugars and fluorophores may be coupled to lectins in such a manner that nucleotides may be fluorescently detected.

### Example 2. Coupling sugars to beads

### Step A. Preparing amino-functionalized alkyl sugars

The alkyl sugar β-GlcNAc-PEG3-Alkyne (SMB00414 from Sigma) is reacted with azido-PEG2-amine as indicated below, using a copper(I) assisted click reaction performed in the presence of copper(II) sulfate with tris(3-hydroxypropyltriazolmethyl)amine (THPTA) as a ligand and sodium ascorbate as a reducing agent, to convert the alkyne group to an amino group and obtain β-mannose-PEG5-amine.

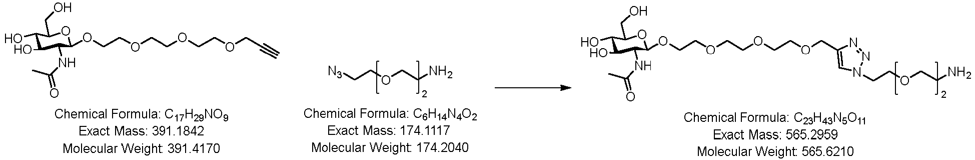

The alkyl sugar α-Man-PEG3-Alkyne (SMB00415 from Sigma) is reacted with azido-PEG2-amine as indicated below, using a copper(I) assisted click reaction performed in the presence of copper(II) sulfate with tris(3-hydroxypropyltriazolmethyl)amine (THPTA) as a ligand and sodium ascorbate as a reducing agent, to convert the alkyne group to an amino group and obtain α-mannose-PEG5-amine.

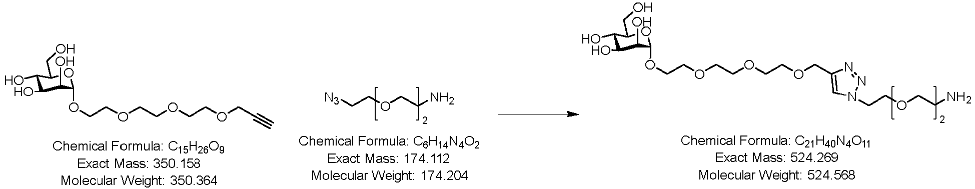

### Step B. Reacting amino-functionalized alkyl sugars with carboxylate-modified beads

Carboxylate-modified, fluorescent polystyrene 20 nm microspheres (FluoSpheres^{®} beads) are obtained from ThermoFisher Scientific. A first set of the microspheres are green, having an excitation maximum at 535 nm and an emission maximum at 575 nm (F8784), while a second set of the microspheres are red, having an excitation maximum at 660 nm and an emission maximum at 680 nm (F8783).

FIG. 9 schematically illustrates an example process flow for coupling sugars to a bead. Carboxylate-modified red microspheres 910 are reacted with the amine-functionalized alkyl mannose of step A to obtain mannose-functionalized red microspheres 920. More specifically, the carboxylate-modified red microspheres (7.5 nmol) and amine-functionalized alkyl mannose (600 nmol) are mixed in a glass vial with 500 mM MES buffer (100 uL, pH 6) and incubated at room temperature for 15 minutes. EDC.HCl (30 umol) in water is added to the mixture and incubated for 15 minutes. The pH is adjusted with 20x borate buffer to pH 8.3. The mixture is rotated overnight at room temperature. The mixture then is quenched with 100 mM Tris buffer (50 uL), and subsequently purified by dialysis. The mannose-functionalized red microspheres resulting from operation 920 are used to fluorescently label the lectin Con A in the manner illustrated in FIG. 5C.

The carboxylate-modified green microspheres are similarly reacted with the amine-functionalized alkyl GlcNAc of step A to obtain GlcNAc-functionalized green microspheres. The GlcNAc-functionalized green microspheres are used to fluorescently label the lectin WGA in the manner illustrated in FIG. 5C. From this example it may be understood that sugars and fluorophores may be coupled to lectins in such a manner that nucleotides may be fluorescently detected.

### Example 3. Preparation of nucleotide-sugar complex

In a non-limiting working example, the azide-functionalized nucleotide ddCTP-N₃ was reacted with alkyne-functionalized alkyl sugar to obtain a nucleotide-sugar complex using the reaction scheme below:

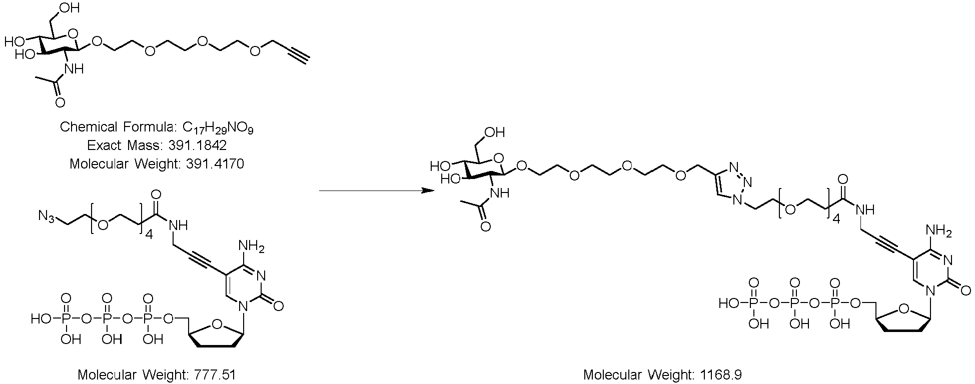

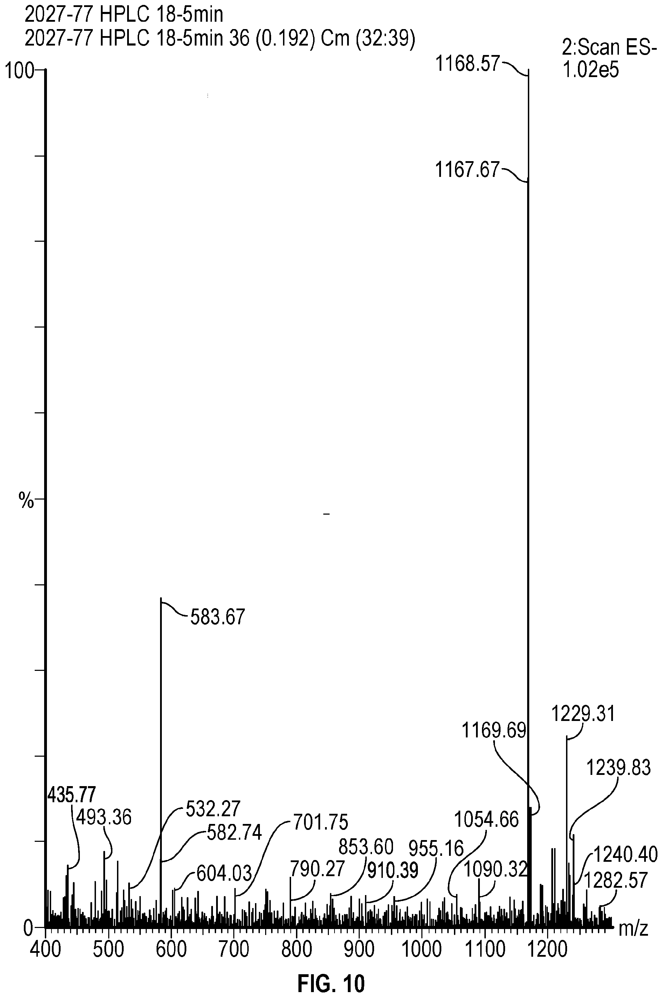

More specifically, 1M stock solutions of each CuSO₄, THPTA, and sodium ascorbate in water were prepared. CuSO₄ (3 uL, 3 umol) and THPTA (9 uL, 9 umol) were pipetted into a tube. Sodium ascorbate (15 uL, 15 umol) was added to the mixture. ddCTP-N₃ (60 nmol, 12uL, 5 mM) and GlcNAc (72 nmol, 14.4 uL, 5 mM) were added to the mixture. 10x PBS (6 uL) was added and the mixture stirred overnight. The product then was purified by HPLC and analyzed on LCMS (negative mode ESI). Calcd. [M-H⁺]⁻ = 1167.9, [M-2H⁺]²⁻ = 583.4. Found [M-H⁺]⁻ = 1167.7, [M-2H⁺]²⁻ = 583.7. FIG. 10 is a liquid chromatography mass spectrometry (LCMS) spectrum of the resulting example nucleotide-sugar complex. Strong peaks were observed at 583.67, 1167.67, and 1168.57 indicating that the nucleotide-sugar complex was successfully prepared.

### Example 4. Characterization of nucleotide-sugar complexes

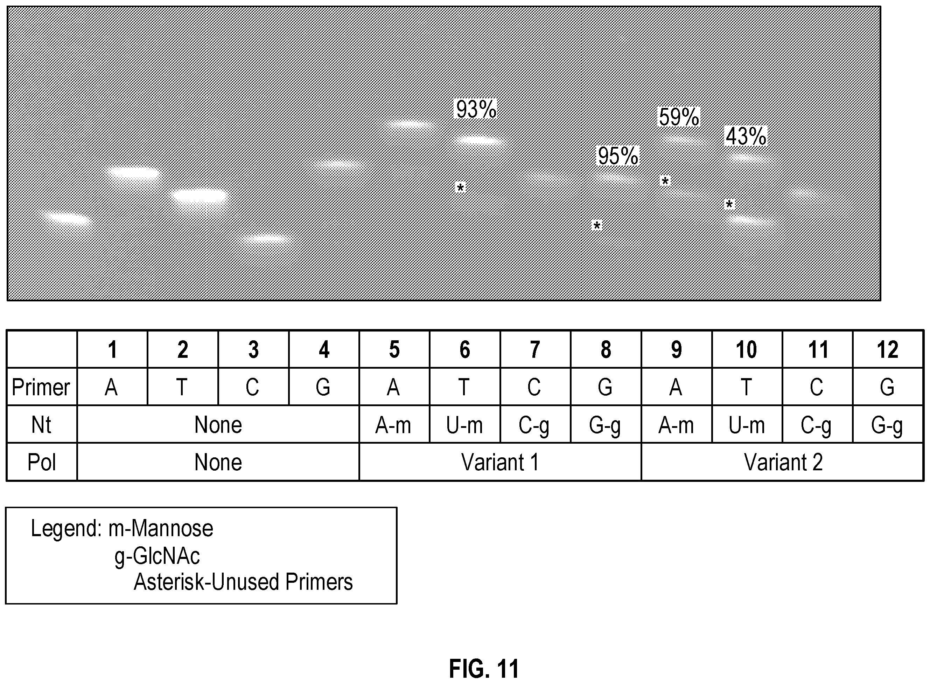

FIG. 11 is an image of a gel showing the results of incorporation of nucleotide-sugar complexes, into growing polynucleotides by polymerases. More specifically, ddATP-mannose, ddUTP-mannose, ddCTP-GlcNAc, and ddGTP-GlcNAc complexes were prepared in a manner such as described in Example 3, and were incorporated into growing polynucleotides using fluorescene amidite (FAM) labeled primers using either a first polymerase ("Variant 1") or a second polymerase ("Variant 2"). In the gel shown in FIG. 11, lanes 1-4 correspond to negative controls, namely primers to which the nucleotide-sugar complexes were not added; lanes 5-8 correspond to incorporation into the primers of ddATP-mannose, ddUTP-mannose, ddCTP-GlcNAc, and ddGTP-GlcNAc complexes using the first polymerase; and lanes 9-12 correspond to incorporation into the primers of ddATP-mannose, ddUTP-mannose, ddCTP-GlcNAc, and ddGTP-GlcNAc complexes using the second polymerase. From comparison of lanes 1, 5, and 9 in FIG. 11, it may be understood that ddATP-mannose successfully became incorporated into the primers using both polymerases. From comparison of lanes 2, 6, and 10 in FIG. 11, it may be understood that ddATP-mannose successfully became incorporated into the primers using both polymerases. From comparison of lanes 3, 7, and 11 in FIG. 11, it may be understood that ddATP-mannose successfully became incorporated into the primers using both polymerases. From comparison of lanes 4, 8, and 12 in FIG. 11, it may be understood that ddATP-mannose successfully became incorporated into the primers using both polymerases. Accordingly, it was demonstrated that ddNTP-sugar complexes may be incorporated into growing polynucleotides.

### Additional notes

It is to be understood that any respective features/examples of each of the aspects of the disclosure as described herein may be implemented together in any appropriate combination, and that any features/examples from any one or more of these aspects may be implemented together with any of the features of the other aspect(s) as described herein in any appropriate combination to achieve the benefits as described herein.

## Claims

1. A method for detecting an element, the method comprising:
coupling a first element-sugar complex to a first substrate, the first element-sugar complex comprising a first element coupled to a first sugar;
coupling a first lectin to the first sugar; and
detecting the first element using at least detection of the first lectin wherein the first element comprises an analyte, the analyte comprising a first nucleotide;
wherein coupling the first element-sugar complex to the first substrate comprises incorporating the first nucleotide into a first oligonucleotide coupled to the first substrate;
wherein the first oligonucleotide is hybridized to a second oligonucleotide, and wherein incorporating the first nucleotide into the first oligonucleotide comprises extending the first oligonucleotide using at least a sequence of the second oligonucleotide.

2. The method of claim 1, wherein coupling the first element-sugar complex to the first substrate comprises flowing a solution over the substrate, the solution comprising:
the first element-sugar complex; and
a second element-sugar complex comprising a second element coupled to a second sugar,
wherein the first lectin selectively binds the first sugar and does not bind the second sugar;
optionally wherein:
(i) the first and second elements comprise different nucleotide types than one another; and/or
(ii) the first and second sugars comprise different sugar types than one another.

3. The method of claims 2 , further comprising:
coupling the second element-sugar complex to the first substrate or to a second substrate;
coupling a second lectin to the second sugar; and
detecting the second element using at least detection of the second lectin; optionally wherein the solution further comprises:
a third element-sugar complex comprising a third element coupled to a third sugar; and
a fourth element-sugar complex comprising a fourth element coupled to a fourth sugar, the method further comprising:
coupling the third element-sugar complex to the first substrate, to the second substrate, or to a third substrate;
coupling a third lectin to the third sugar;
detecting the third element using at least detection of the third lectin;
coupling the fourth element-sugar complex to the first substrate, to the second substrate, to the third substrate, or to a fourth substrate;
coupling a fourth lectin to the fourth sugar; and
detecting the fourth element using at least detection of the fourth lectin.

4. The method of any one of claims 1 to 3, wherein:
(i) the first element sugar-complex further comprises a fifth sugar coupled to the element; optionally wherein the method further comprises coupling the first lectin to the fifth sugar.

5. The method of any one of claims 1 to 4, further comprising coupling a first fluorophore to the first lectin, wherein detection of the first lectin comprises detecting fluorescence from the first fluorophore; optionally wherein
the first fluorophore comprises a first plurality of fluorophores.

6. The method of claim 5, wherein coupling the first fluorophore to the first lectin comprises coupling a polymer to the first lectin, wherein the polymer comprises the first fluorophore and a sixth sugar, wherein the first lectin couples to the sixth sugar;
optionally wherein the polymer comprises a bead or a polypeptide.

7. The method of claim 5 or claim 6, wherein:
(i) the first fluorophore is coupled to the first lectin before coupling the first lectin to the first sugar; or
(ii) the first fluorophore is coupled to the first lectin after coupling the first lectin to the first sugar.

8. The method of claim 6, wherein:
a seventh sugar is coupled to the first lectin,
a second lectin is coupled to the seventh sugar, and
detection of the first lectin comprises detection of the second lectin; optionally wherein a second fluorophore is coupled to the second lectin, and wherein detection of the first lectin comprises detecting fluorescence from the second fluorophore; optionally wherein the first fluorophore is a different type of fluorophore than the second fluorophore.

9. The method of claim 8, wherein the first lectin comprises Concanavalin A (Con A), the seventh sugar comprises N-acetyl-galactosamine (GalNAc), and the second lectin comprises soybean agglutinin (SBA).

10. The method of any one of claims 1 to 8, wherein:
the first lectin comprises Concanavalin A (Con A) and the first sugar comprises mannose (Man) or glucose (Glc);
the first lectin comprises wheat germ agglutinin (WGA) and the first sugar comprises N-acetyl-glucosamine (GlcNAc) or N-acetyl-neuraminic acid (Neu5Ac);
the first lectin comprises soybean agglutinin (SBA) and the first sugar comprises galactose (Gal) or N-acetyl-galactosamine (GalNAc);
the first lectin comprises *Dolichos bifloris* (DBA) and the first sugar comprises GalNAcα3GalNAc or GalNAc;
the first lectin comprises Ricin and the first sugar comprises galactose;
the first lectin comprises peanut agglutinin (PNA) and the first sugar comprises galactose or Galβ3GalNAcα (T-antigen);
the first lectin comprises *Pisum sativum* (PSA) and the first sugar comprises mannose or glucose;
the first lectin comprises *Lens culinaris* (LCA) and the first sugar comprises mannose or glucose;
the first lectin comprises *Galanthus nivalus* (GNA) and the first sugar comprises mannose;
the first lectin comprises *Solanum tuberosum* (STA) and the first sugar comprises (GlcNAc)ₙ;
the first lectin comprises Asialoglycoprotein receptor (ASGPR) H1 and the first sugar comprises galactose;
the first lectin comprises Galectin-3 and the first sugar comprises galactose;
the first lectin comprises Sialoadhesin and the first sugar comprises Neu5Ac;
the first lectin comprises Cation-dependent mannose-6-phosphate receptor (CD-MPR) and the first sugar comprises Man6P; or
the first lectin comprises C-reactive protein (CRP) and the first sugar comprises galactose, Gal6P, or galacturonic acid.

11. The method of any one of claims 1 to 10, wherein:
(i) the first substrate comprises a bead; and/or
(ii) the first sugar comprises an alkyl sugar; and/or
(iii) the first sugar comprises a monosaccharide; and/or
(iv) the first sugar comprises a disaccharide.

12. A system, comprising:
a substrate;
a first element-sugar complex coupled to the substrate, the first element-sugar complex comprising a first element coupled to a first sugar;
a first lectin coupled to the first sugar; and
detection circuitry to detect the first element using at least detection of the first lectin;
wherein the first element comprises an analyte comprising a first nucleotide;
wherein the first element-sugar complex is coupled to the first substrate via incorporation of the first nucleotide into a first oligonucleotide coupled to the first substrate;
wherein the first oligonucleotide is hybridized to a second oligonucleotide, and wherein the incorporation of the first nucleotide into the first oligonucleotide comprises extending the first oligonucleotide using at least a sequence of the second oligonucleotide.

13. The system of claim 12, further comprising a solution to flow over the substrate, the solution comprising:
the first element-sugar complex; and
a second element-sugar complex comprising a second element coupled to a second sugar,
wherein the first lectin selectively binds the first sugar and does not bind the second sugar;
optionally wherein:
(i) the first and second elements comprise different nucleotide types than one another; and/or
(ii) the first and second sugars comprise different sugar types than one another.

14. The system of claims 13, wherein:
the second element-sugar complex is coupled to the first substrate or to a second substrate;
a second lectin is coupled to the second sugar; and
the detection circuitry is to detect the second element using at least detection of the second lectin;
optionally wherein:
the solution further comprises:
a third element-sugar complex comprising a third element coupled to a third sugar, and a fourth element-sugar complex comprising a fourth element coupled to a fourth sugar;
the third element-sugar complex is coupled to the first substrate, to the second substrate, or to a third substrate;
a third lectin is coupled to the third sugar;
the detection circuitry is to detect the third element using at least detection of the third lectin;
the fourth element-sugar complex is coupled to the first substrate, to the second substrate, to the third substrate, or to a fourth substrate;
a fourth lectin is coupled to the fourth sugar; and
the detection circuitry is to detect the fourth element using at least detection of the fourth lectin.

15. The system of claim 14, wherein the first element sugar-complex further comprises a fifth sugar coupled to the element; optionally wherein the first lectin is coupled to the fifth sugar.

16. The system of any one of claims 12 to 15, wherein a first fluorophore is coupled to the first lectin, wherein the detection circuitry is to detect the first lectin using at least detection of fluorescence from the first fluorophore;
optionally wherein the first fluorophore comprises a first plurality of fluorophores.

17. The system of claim 15, wherein the first fluorophore is coupled to the first lectin using a polymer, wherein the polymer comprises the first fluorophore and a sixth sugar,
wherein the first lectin couples to the sixth sugar;
optionally wherein the polymer comprises a bead or a polypeptide.

18. The system of claim 16 or claim 17, wherein:
(i) the first fluorophore is coupled to the first lectin before the first lectin is coupled to the first sugar; or
(ii) the first fluorophore is coupled to the first lectin after the first lectin is coupled to the first sugar.

19. The system of claim 17, wherein:
a seventh sugar is coupled to the first lectin,
a second lectin is coupled to the seventh sugar, and
the detection circuitry is to detect the first lectin using at least detection of the second lectin;
optionally wherein a second fluorophore is coupled to the second lectin, and wherein the detection circuitry is to detect the first lectin using at least detecting fluorescence from the second fluorophore, optionally wherein the first fluorophore is a different type of fluorophore than the second fluorophore.

20. The system of claim 19, wherein the first lectin comprises Concanavalin A (Con A), the seventh sugar comprises N-acetyl-galactosamine (GalNAc), and the second lectin comprises soybean agglutinin (SBA).

21. The system of any one of claims 12 to 19, wherein:
the first lectin comprises Concanavalin A (Con A) and the first sugar comprises mannose (Man) or glucose (Glc);
the first lectin comprises wheat germ agglutinin (WGA) and the first sugar comprises N-acetyl-glucosamine (GlcNAc) or N-acetyl-neuraminic acid (Neu5Ac);
the first lectin comprises soybean agglutinin (SBA) and the first sugar comprises galactose (Gal) or N-acetyl-galactosamine (GalNAc);
the first lectin comprises *Dolichos bifloris* (DBA) and the first sugar comprises GalNAcα3GalNAc or GalNAc;
the first lectin comprises Ricin and the first sugar comprises galactose;
the first lectin comprises peanut agglutinin (PNA) and the first sugar comprises galactose or Galβ3GalNAcα (T-antigen);
the first lectin comprises *Pisum sativum* (PSA) and the first sugar comprises mannose or glucose;
the first lectin comprises *Lens culinaris* (LCA) and the first sugar comprises mannose or glucose;
the first lectin comprises *Galanthus nivalus* (GNA) and the first sugar comprises mannose;
the first lectin comprises *Solanum tuberosum* (STA) and the first sugar comprises (GlcNAc)ₙ;
the first lectin comprises Asialoglycoprotein receptor (ASGPR) H1 and the first sugar comprises galactose;
the first lectin comprises Galectin-3 and the first sugar comprises galactose;
the first lectin comprises Sialoadhesin and the first sugar comprises Neu5Ac;
the first lectin comprises Cation-dependent mannose-6-phosphate receptor (CD-MPR) and the first sugar comprises Man6P; or
the first lectin comprises C-reactive protein (CRP) and the first sugar comprises galactose, Gal6P, or galacturonic acid.

22. The system of any one of claims 12 to 21, wherein:
(i) the first substrate comprises a bead; and/or (ii) the first sugar comprises an alkyl sugar; and/or
(iii) the first sugar comprises a monosaccharide; and/or
(iv) the first sugar comprises a disaccharide.

## Patentansprüche

1. Ein Verfahren zum Nachweisen eines Elements, wobei das Verfahren Folgendes beinhaltet:
Koppeln eines ersten Element-Zucker-Komplexes mit einem ersten Substrat, wobei der erste Element-Zucker-Komplex ein erstes Element, das mit einem ersten Zucker gekoppelt ist, beinhaltet;
Koppeln eines ersten Lektins mit dem ersten Zucker; und
Nachweisen des ersten Elements unter Verwendung mindestens des Nachweises des ersten Lektins, wobei das erste Element einen Analyten beinhaltet, wobei der Analyt ein erstes Nukleotid beinhaltet;
wobei das Koppeln des ersten Element-Zucker-Komplexes mit dem ersten Substrat das Einbauen des ersten Nukleotids in ein erstes Oligonukleotid, das mit dem ersten Substrat gekoppelt ist, beinhaltet;
wobei das erste Oligonukleotid an ein zweites Oligonukleotid hybridisiert wird und
wobei das Einbauen des ersten Nukleotids in das erste Oligonukleotid das Verlängern des ersten Oligonukleotids unter Verwendung von mindestens einer Sequenz des zweiten Oligonukleotids beinhaltet.

2. Verfahren gemäß Anspruch 1, wobei das Koppeln des ersten Element-Zucker-Komplexes mit dem ersten Substrat das Fließenlassen einer Lösung über das Substrat beinhaltet, wobei die Lösung Folgendes beinhaltet:
den ersten Element-Zucker-Komplex; und
einen zweiten Element-Zucker-Komplex, der ein zweites Element, das mit einem zweiten Zucker gekoppelt ist, beinhaltet,
wobei das erste Lektin selektiv den ersten Zucker bindet und den zweiten Zucker nicht bindet;
wobei optional:
(i) das erste und zweite Element voneinander verschiedene Nukleotidtypen beinhalten; und/oder
(ii) der erste und zweite Zucker voneinander verschiedene Zuckertypen beinhalten.

3. Verfahren gemäß Anspruch 2, das ferner Folgendes beinhaltet:
Koppeln des zweiten Element-Zucker-Komplexes mit dem ersten Substrat oder mit einem zweiten Substrat;
Koppeln eines zweiten Lektins mit dem zweiten Zucker; und
Nachweisen des zweiten Elements unter Verwendung mindestens des Nachweises des zweiten Lektins; wobei die Lösung optional ferner Folgendes beinhaltet:
einen dritten Element-Zucker-Komplex, der ein drittes Element, das mit einem dritten Zucker gekoppelt ist, beinhaltet; und
einen vierten Element-Zucker-Komplex, der ein viertes Element, das mit einem vierten Zucker gekoppelt ist, beinhaltet, wobei das Verfahren ferner Folgendes beinhaltet:
Koppeln des dritten Element-Zucker-Komplexes mit dem ersten Substrat, mit dem zweiten Substrat oder mit einem dritten Substrat;
Koppeln eines dritten Lektins mit dem dritten Zucker;
Nachweisen des dritten Elements unter Verwendung mindestens des Nachweises des dritten Lektins;
Koppeln des vierten Element-Zucker-Komplexes mit dem ersten Substrat, mit dem zweiten Substrat, mit dem dritten Substrat oder mit einem vierten Substrat;
Koppeln eines vierten Lektins mit dem vierten Zucker; und
Nachweisen des vierten Elements unter Verwendung mindestens des Nachweises des vierten Lektins.

4. Verfahren gemäß einem der Ansprüche 1 bis 3, wobei:
(i) der erste Element-Zucker-Komplex ferner einen fünften Zucker, der mit dem Element gekoppelt ist, beinhaltet; wobei das Verfahren optional ferner das Koppeln des ersten Lektins mit dem fünften Zucker beinhaltet.

5. Verfahren gemäß einem der Ansprüche 1 bis 4, das ferner das Koppeln eines ersten Fluorophors mit dem ersten Lektin beinhaltet, wobei der Nachweis des ersten Lektins das Nachweisen von Fluoreszenz von dem ersten Fluorophor beinhaltet; wobei der erste Fluorophor optional eine erste Vielzahl von Fluorophoren beinhaltet.

6. Verfahren gemäß Anspruch 5, wobei das Koppeln des ersten Fluorophors mit dem ersten Lektin das Koppeln eines Polymers mit dem ersten Lektin beinhaltet, wobei das Polymer den ersten Fluorophor und einen sechsten Zucker beinhaltet, wobei das erste Lektin sich an den sechsten Zucker koppelt; wobei das Polymer optional ein Kügelchen oder ein Polypeptid beinhaltet.

7. Verfahren gemäß Anspruch 5 oder Anspruch 6, wobei:
(i) der erste Fluorophor mit dem ersten Lektin gekoppelt wird, bevor das erste Lektin mit dem ersten Zucker gekoppelt wird; oder
(ii) der erste Fluorophor mit dem ersten Lektin gekoppelt wird, nachdem das erste Lektin mit dem ersten Zucker gekoppelt wurde.

8. Verfahren gemäß Anspruch 6, wobei:
ein siebter Zucker mit dem ersten Lektin gekoppelt wird,
ein zweites Lektin mit dem siebten Zucker gekoppelt wird und
der Nachweis des ersten Lektins den Nachweis des zweiten Lektins beinhaltet; wobei ein zweiter Fluorophor optional mit dem zweiten Lektin gekoppelt ist, und wobei der Nachweis des ersten Lektins das Nachweisen von Fluoreszenz von dem zweiten Fluorophor beinhaltet;
wobei der erste Fluorophor optional ein anderer Typ Fluorophor ist als der zweite Fluorophor.

9. Verfahren gemäß Anspruch 8, wobei das erste Lektin Concanavalin A (Con A) beinhaltet, der siebte Zucker N-Acetylgalactosamin (GalNAc) beinhaltet und das zweite Lektin Sojabohnenagglutinin (SBA) beinhaltet.

10. Verfahren gemäß einem der Ansprüche 1 bis 8, wobei:
das erste Lektin Concanavalin A (Con A) beinhaltet und der erste Zucker Mannose (Man) oder Glukose (Glc) beinhaltet;
das erste Lektin Weizenkeimagglutinin (WGA) beinhaltet und der erste Zucker N-Acetylglucosamin (GlcNAc) oder N-Acetylneuraminsäure (Neu5Ac) beinhaltet;
das erste Lektin Sojabohnenagglutinin (SBA) beinhaltet und der erste Zucker Galaktose (Gal) oder N-Acetylgalactosamin (GalNAc) beinhaltet;
das erste Lektin *Dolichos bifloris* (DBA) beinhaltet und der erste Zucker GalNAcα3GalNAc oder GalNAc beinhaltet;
das erste Lektin Ricin beinhaltet und der erste Zucker Galaktose beinhaltet;
das erste Lektin Erdnussagglutinin (PNA) beinhaltet und der erste Zucker Galaktose oder Galβ3GalNAcα (T-Antigen) beinhaltet;
das erste Lektin *Pisum sativum* (PSA) beinhaltet und der erste Zucker Mannose oder Glukose beinhaltet;
das erste Lektin *Lens culinaris* (LCA) beinhaltet und der erste Zucker Mannose oder Glukose beinhaltet;
das erste Lektin *Galanthus nivalis* (GNA) beinhaltet und der erste Zucker Mannose beinhaltet;
das erste Lektin *Solanum tuberosum* (STA) beinhaltet und der erste Zucker (GlcNAc)ₙ beinhaltet;
das erste Lektin Asialoglycoprotein-Rezeptor (ASGPR) H1 beinhaltet und der erste Zucker Galaktose beinhaltet;
das erste Lektin Galektin-3 beinhaltet und der erste Zucker Galaktose beinhaltet;
das erste Lektin Sialoadhäsin beinhaltet und der erste Zucker Neu5Ac beinhaltet;
das erste Lektin kationenabhängigen Mannose-6-phosphat-Rezeptor (CD-MPR) beinhaltet und der erste Zucker Man6P beinhaltet; oder
das erste Lektin C-reaktives Protein (CRP) beinhaltet und der erste Zucker Galaktose, Gal6P oder Galakturonsäure beinhaltet.

11. Verfahren gemäß einem der Ansprüche 1 bis 10, wobei:
(i) das erste Substrat ein Kügelchen beinhaltet; und/oder
(ii) der erste Zucker einen Alkylzucker beinhaltet; und/oder
(iii) der erste Zucker ein Monosaccharid beinhaltet; und/oder
(iv) der erste Zucker ein Disaccharid beinhaltet.

12. Ein System, das Folgendes beinhaltet:
ein Substrat;
einen ersten Element-Zucker-Komplex, der mit dem Substrat gekoppelt wird, wobei der erste Element-Zucker-Komplex ein erstes Element, das mit einem ersten Zucker gekoppelt ist, beinhaltet;
ein erstes Lektin, das mit dem ersten Zucker gekoppelt wird; und
Nachweisschaltungen, um das erste Element unter Verwendung mindestens des Nachweises des ersten Lektins nachzuweisen;
wobei das erste Element einen Analyten beinhaltet, der ein erstes Nukleotid beinhaltet; wobei der erste Element-Zucker-Komplex mit dem ersten Substrat mittels Einbau des ersten Nukleotids in ein erstes Oligonukleotid, das mit dem ersten Substrat gekoppelt ist, gekoppelt wird;
wobei das erste Oligonukleotid an ein zweites Oligonukleotid hybridisiert wird und
wobei der Einbau des ersten Nukleotids in das erste Oligonukleotid das Verlängern des ersten Oligonukleotids unter Verwendung mindestens einer Sequenz des zweiten Oligonukleotids beinhaltet.

13. System gemäß Anspruch 12, das ferner eine Lösung zum Fließen über das Substrat beinhaltet, wobei die Lösung Folgendes beinhaltet:
den ersten Element-Zucker-Komplex; und
einen zweiten Element-Zucker-Komplex, der ein zweites Element, das mit einem zweiten Zucker gekoppelt ist, beinhaltet,
wobei das erste Lektin selektiv den ersten Zucker bindet und den zweiten Zucker nicht bindet;
wobei optional:
(i) das erste und zweite Element voneinander verschiedene Nukleotidtypen beinhalten; und/oder
(ii) der erste und zweite Zucker voneinander verschiedene Zuckertypen beinhalten.

14. System gemäß Anspruch 13, wobei:
der zweite Element-Zucker-Komplex mit dem ersten Substrat oder mit einem zweiten Substrat gekoppelt wird;
ein zweites Lektin mit dem zweiten Zucker gekoppelt wird; und
die Nachweisschaltungen dazu dienen, das zweite Element unter Verwendung mindestens des Nachweises des zweiten Lektins nachzuweisen;
wobei optional:
die Lösung ferner Folgendes beinhaltet:
einen dritten Element-Zucker-Komplex, der ein drittes Element, das mit einem dritten Zucker gekoppelt ist, beinhaltet, und einen vierten Element-Zucker-Komplex, der ein viertes Element, das mit einem vierten Zucker gekoppelt ist, beinhaltet;
der dritte Element-Zucker-Komplex mit dem ersten Substrat, mit dem zweiten Substrat oder mit einem dritten Substrat gekoppelt wird;
ein drittes Lektin mit dem dritten Zucker gekoppelt wird;
die Nachweisschaltungen dazu dienen, das dritte Element unter Verwendung mindestens des Nachweises des dritten Lektins nachzuweisen;
der vierte Element-Zucker-Komplex mit dem ersten Substrat, mit dem zweiten Substrat, mit dem dritten Substrat oder mit einem vierten Substrat gekoppelt wird;
ein viertes Lektin mit dem vierten Zucker gekoppelt wird; und
die Nachweisschaltungen dazu dienen, das vierte Element unter Verwendung mindestens des Nachweises des vierten Lektins nachzuweisen.

15. System gemäß Anspruch 14, wobei der erste Element-Zucker-Komplex ferner einen fünften Zucker, der mit dem Element gekoppelt ist, beinhaltet;
wobei das erste Lektin optional mit dem fünften Zucker gekoppelt wird.

16. System gemäß einem der Ansprüche 12 bis 15, wobei ein erster Fluorophor mit dem ersten Lektin gekoppelt ist, wobei die Nachweisschaltungen dazu dienen, das erste Lektin unter Verwendung mindestens des Nachweises von Fluoreszenz von dem ersten Fluorophor nachzuweisen;
wobei der erste Fluorophor optional eine erste Vielzahl von Fluorophoren beinhaltet.

17. System gemäß Anspruch 15, wobei der erste Fluorophor unter Verwendung eines Polymers mit dem ersten Lektin gekoppelt ist, wobei das Polymer den ersten Fluorophor und einen sechsten Zucker beinhaltet, wobei das erste Lektin sich mit dem sechsten Zucker koppelt;
wobei das Polymer optional ein Kügelchen oder ein Polypeptid beinhaltet.

18. System gemäß Anspruch 16 oder Anspruch 17, wobei:
(i) der erste Fluorophor mit dem ersten Lektin gekoppelt wird, bevor das erste Lektin mit dem ersten Zucker gekoppelt wird; oder
(ii) der erste Fluorophor mit dem ersten Lektin gekoppelt wird, nachdem das erste Lektin mit dem ersten Zucker gekoppelt wurde.

19. System gemäß Anspruch 17, wobei:
ein siebter Zucker mit dem ersten Lektin gekoppelt wird,
ein zweites Lektin mit dem siebten Zucker gekoppelt wird und
die Nachweisschaltungen dazu dienen, das erste Lektin unter Verwendung mindestens des Nachweises des zweiten Lektins nachzuweisen;
wobei ein zweiter Fluorophor optional mit dem zweiten Lektin gekoppelt ist und wobei die Nachweisschaltungen dazu dienen, das erste Lektin unter Verwendung mindestens des Nachweisens von Fluoreszenz von dem zweiten Fluorophor nachzuweisen, wobei der erste Fluorophor optional ein anderer Typ Fluorophor ist als der zweite Fluorophor.

20. System gemäß Anspruch 19, wobei das erste Lektin Concanavalin A (Con A) beinhaltet, der siebte Zucker N-Acetylgalactosamin (GalNAc) beinhaltet und das zweite Lektin Sojabohnenagglutinin (SBA) beinhaltet.

21. System gemäß einem der Ansprüche 12 bis 19, wobei:
das erste Lektin Concanavalin A (Con A) beinhaltet und der erste Zucker Mannose (Man) oder Glukose (Glc) beinhaltet;
das erste Lektin Weizenkeimagglutinin (WGA) beinhaltet und der erste Zucker N-Acetylglucosamin (GlcNAc) oder N-Acetylneuraminsäure (Neu5Ac) beinhaltet;
das erste Lektin Sojabohnenagglutinin (SBA) beinhaltet und der erste Zucker Galaktose (Gal) oder N-Acetylgalactosamin (GalNAc) beinhaltet;
das erste Lektin *Dolichos bifloris* (DBA) beinhaltet und der erste Zucker GalNAcα3GalNAc oder GalNAc beinhaltet;
das erste Lektin Ricin beinhaltet und der erste Zucker Galaktose beinhaltet;
das erste Lektin Erdnussagglutinin (PNA) beinhaltet und der erste Zucker Galaktose oder Galβ3GalNAcα (T-Antigen) beinhaltet;
das erste Lektin *Pisum sativum* (PSA) beinhaltet und der erste Zucker Mannose oder Glukose beinhaltet;
das erste Lektin *Lens culinaris* (LCA) beinhaltet und der erste Zucker Mannose oder Glukose beinhaltet;
das erste Lektin *Galanthus nivalus* (GNA) beinhaltet und der erste Zucker Mannose beinhaltet;
das erste Lektin *Solanum tuberosum* (STA) beinhaltet und der erste Zucker (GlcNAc)ₙ beinhaltet;
das erste Lektin Asialoglycoprotein-Rezeptor (ASGPR) H1 beinhaltet und der erste Zucker Galaktose beinhaltet;
das erste Lektin Galektin-3 beinhaltet und der erste Zucker Galaktose beinhaltet;
das erste Lektin Sialoadhäsin beinhaltet und der erste Zucker Neu5Ac beinhaltet;
das erste Lektin kationenabhängigen Mannose-6-phosphat-Rezeptor (CD-MPR) beinhaltet und der erste Zucker Man6P beinhaltet; oder
das erste Lektin C-reaktives Protein (CRP) beinhaltet und der erste Zucker Galaktose, Gal6P oder Galakturonsäure beinhaltet.

22. System gemäß einem der Ansprüche 12 bis 21, wobei:
(i) das erste Substrat ein Kügelchen beinhaltet; und/oder
(ii) der erste Zucker einen Alkylzucker beinhaltet; und/oder
(iii) der erste Zucker ein Monosaccharid beinhaltet; und/oder
(iv) der erste Zucker ein Disaccharid beinhaltet.

## Revendications

1. Un procédé pour la détection d'un élément, le procédé comprenant :
le fait de coupler un premier complexe élément-sucre à un premier substrat, le premier complexe élément-sucre comprenant un premier élément couplé à un premier sucre ;
le fait de coupler une première lectine au premier sucre ; et
le fait de détecter le premier élément en utilisant au moins la détection de la première lectine où le premier élément comprend un analyte, l'analyte comprenant un premier nucléotide ;
où le fait de coupler le premier complexe élément-sucre au premier substrat comprend de fait d'incorporer le premier nucléotide dans un premier oligonucléotide couplé au premier substrat ;
où le premier oligonucléotide est hybridé à un deuxième oligonucléotide, et où le fait d'incorporer le premier nucléotide dans le premier oligonucléotide comprend le fait d'étendre le premier oligonucléotide en utilisant au moins une séquence du deuxième oligonucléotide.

2. Le procédé de la revendication 1, où le fait de coupler le premier complexe élément-sucre au premier substrat comprend le fait de faire s'écouler une solution par-dessus le substrat, la solution comprenant :
le premier complexe élément-sucre ; et
un deuxième complexe élément-sucre comprenant un deuxième élément couplé à un deuxième sucre,
où la première lectine se lie sélectivement au premier sucre et ne se lie pas au deuxième sucre ;
facultativement où :
(i) les premier et deuxième éléments comprennent des types de nucléotides différents l'un de l'autre ; et/ou
(ii) les premier et deuxième sucres comprennent des types de sucres différents l'un de l'autre.

3. Le procédé de la revendication 2, comprenant en sus :
le fait de coupler le deuxième complexe élément-sucre au premier substrat ou à un deuxième substrat ;
le fait de coupler une deuxième lectine au deuxième sucre ; et
le fait de détecter le deuxième élément en utilisant au moins la détection de la deuxième lectine ; facultativement où la solution comprend en sus :
un troisième complexe élément-sucre comprenant un troisième élément couplé à un troisième sucre ; et
un quatrième complexe élément-sucre comprenant un quatrième élément couplé à un quatrième sucre, le procédé comprenant en sus :
le fait de coupler le troisième complexe élément-sucre au premier substrat, au deuxième substrat, ou à un troisième substrat ;
le fait de coupler une troisième lectine au troisième sucre ;
le fait de détecter le troisième élément en utilisant au moins la détection de la troisième lectine ;
le fait de coupler le quatrième complexe élément-sucre au premier substrat, au deuxième substrat, au troisième substrat, ou à un quatrième substrat ;
le fait de coupler une quatrième lectine au quatrième sucre ; et
le fait de détecter le quatrième élément en utilisant au moins la détection de la quatrième lectine.

4. Le procédé de l'une quelconque des revendications 1 à 3, où :
(i) le premier complexe élément-sucre comprend en sus un cinquième sucre couplé à l'élément ; facultativement où le procédé comprend en sus le fait de coupler la première lectine au cinquième sucre.

5. Le procédé de l'une quelconque des revendications 1 à 4, comprenant en sus le fait de coupler un premier fluorophore à la première lectine, où la détection de la première lectine comprend le fait de détecter une fluorescence provenant du premier fluorophore ; facultativement où le premier fluorophore comprend une première pluralité de fluorophores.

6. Le procédé de la revendication 5, où le fait de coupler le premier fluorophore à la première lectine comprend le fait de coupler un polymère à la première lectine, où le polymère comprend le premier fluorophore et un sixième sucre, où la première lectine se couple au sixième sucre ; facultativement où le polymère comprend une bille ou un polypeptide.

7. Le procédé de la revendication 5 ou de la revendication 6, où :
(i) le premier fluorophore est couplé à la première lectine avant le fait de coupler la première lectine au premier sucre ; ou
(ii) le premier fluorophore est couplé à la première lectine après le fait de coupler la première lectine au premier sucre.

8. Le procédé de la revendication 6, où :
un septième sucre est couplé à la première lectine,
une deuxième lectine est couplée au septième sucre, et
la détection de la première lectine comprend la détection de la deuxième lectine ;
facultativement où un deuxième fluorophore est couplé à la deuxième lectine, et où la détection de la première lectine comprend le fait de détecter une fluorescence provenant du deuxième fluorophore ;
facultativement où le premier fluorophore est un type de fluorophore différent du deuxième fluorophore.

9. Le procédé de la revendication 8, où la première lectine comprend la concanavaline A (Con A), le septième sucre comprend la N-acétylgalactosamine (GalNAc), et la deuxième lectine comprend l'agglutinine de soja (SBA).

10. Le procédé de l'une quelconque des revendications 1 à 8, où :
la première lectine comprend la concanavaline A (Con A) et le premier sucre comprend le mannose (Man) ou le glucose (Glc) ;
la première lectine comprend l'agglutinine de germe de blé (WGA) et le premier sucre comprend la N-acétylglucosamine (GlcNAc) ou l'acide N-acétylneuraminique (Neu5Ac) ;
la première lectine comprend l'agglutinine de soja (SBA) et le premier sucre comprend le galactose (Gal) ou la N-acétylgalactosamine (GalNAc) ;
la première lectine comprend la *Dolichos bifloris* (DBA) et le premier sucre comprend GalNAcα3GalNAc ou GalNAc ;
la première lectine comprend la ricine et le premier sucre comprend le galactose ;
la première lectine comprend l'agglutinine d'arachide (PNA) et le premier sucre comprend le galactose ou Galβ3GalNAcα (antigène T) ;
la première lectine comprend la *Pisum sativum* (PSA) et le premier sucre comprend le mannose ou le glucose ;
la première lectine comprend la *Lens culinaris* (LCA) et le premier sucre comprend le mannose ou le glucose ;
la première lectine comprend la *Galanthus nivalis* (GNA) et le premier sucre comprend le mannose ;
la première lectine comprend la *Solanum tuberosum* (STA) et le premier sucre comprend (GlcNAc)ₙ ;
la première lectine comprend le récepteur des asialoglycoprotéines (R-ASGP) H1 et le premier sucre comprend le galactose ;
la première lectine comprend la galectine-3 et le premier sucre comprend le galactose ;
la première lectine comprend la sialoadhésine et le premier sucre comprend Neu5Ac ;
la première lectine comprend le récepteur du mannose-6-phosphate dépendant des cations (CD-MPR) et le premier sucre comprend Man6P ; ou
la première lectine comprend la protéine C-réactive (CPR) et le premier sucre comprend le galactose, Gal6P, ou l'acide galacturonique.

11. Le procédé de l'une quelconque des revendications 1 à 10, où :
(i) le premier substrat comprend une bille ; et/ou
(ii) le premier sucre comprend un alkyl sucre ; et/ou
(iii) le premier sucre comprend un monosaccharide ; et/ou
(iv) le premier sucre comprend un disaccharide.

12. Un système, comprenant :
un substrat ;
un premier complexe élément-sucre couplé au substrat, le premier complexe élément-sucre comprenant un premier élément couplé à un premier sucre ;
une première lectine couplée au premier sucre ; et
des circuits de détection destinés à détecter le premier élément en utilisant au moins la détection de la première lectine ;
où le premier élément comprend un analyte comprenant un premier nucléotide ; où le premier complexe élément-sucre est couplé au premier substrat via l'incorporation du premier nucléotide dans un premier oligonucléotide couplé au premier substrat ;
où le premier oligonucléotide est hybridé à un deuxième oligonucléotide, et où l'incorporation du premier nucléotide dans le premier oligonucléotide comprend le fait d'étendre le premier oligonucléotide en utilisant au moins une séquence du deuxième oligonucléotide.

13. Le système de la revendication 12, comprenant en sus une solution destinée à s'écouler par-dessus le substrat, la solution comprenant :
le premier complexe élément-sucre ; et
un deuxième complexe élément-sucre comprenant un deuxième élément couplé à un deuxième sucre,
où la première lectine se lie sélectivement au premier sucre et ne se lie pas au deuxième sucre ;
facultativement où :
(i) les premier et deuxième éléments comprennent des types de nucléotides différents l'un de l'autre ; et/ou
(ii) les premier et deuxième sucres comprennent des types de sucres différents l'un de l'autre.

14. Le système de la revendication 13, où :
le deuxième complexe élément-sucre est couplé au premier substrat ou à un deuxième substrat ;
une deuxième lectine est couplée au deuxième sucre ; et
les circuits de détection sont destinés à détecter le deuxième élément en utilisant au moins la détection de la deuxième lectine ;
facultativement où :
la solution comprend en sus :
un troisième complexe élément-sucre comprenant un troisième élément couplé à un troisième sucre, et un quatrième complexe élément-sucre comprenant un quatrième élément couplé à un quatrième sucre ;
le troisième complexe élément-sucre est couplé au premier substrat, au deuxième substrat, ou à un troisième substrat ;
une troisième lectine est couplée au troisième sucre ;
les circuits de détection sont destinés à détecter le troisième élément en utilisant au moins la détection de la troisième lectine ;
le quatrième complexe élément-sucre est couplé au premier substrat, au deuxième substrat, au troisième substrat, ou à un quatrième substrat ;
une quatrième lectine est couplée au quatrième sucre ; et
les circuits de détection sont destinés à détecter le quatrième élément en utilisant au moins la détection de la quatrième lectine.

15. Le système de la revendication 14, où le premier complexe élément-sucre comprend en sus un cinquième sucre couplé à l'élément ;
facultativement où la première lectine est couplée au cinquième sucre.

16. Le système de l'une quelconque des revendications 12 à 15, où un premier fluorophore est couplé à la première lectine, où les circuits de détection sont destinés à détecter la première lectine en utilisant au moins la détection d'une fluorescence provenant du premier fluorophore ;
facultativement où le premier fluorophore comprend une première pluralité de fluorophores.

17. Le système de la revendication 15, où le premier fluorophore est couplé à la première lectine en utilisant un polymère, où le polymère comprend le premier fluorophore et un sixième sucre, où la première lectine se couple au sixième sucre ;
facultativement où le polymère comprend une bille ou un polypeptide.

18. Le système de la revendication 16 ou de la revendication 17, où :
(i) le premier fluorophore est couplé à la première lectine avant que la première lectine soit couplée au premier sucre ; ou
(ii) le premier fluorophore est couplé à la première lectine après que la première lectine a été couplée au premier sucre.

19. Le système de la revendication 17, où :
un septième sucre est couplé à la première lectine,
une deuxième lectine est couplée au septième sucre, et
les circuits de détection sont destinés à détecter la première lectine en utilisant au moins la détection de la deuxième lectine ;
facultativement où un deuxième fluorophore est couplé à la deuxième lectine, et où les circuits de détection sont destinés à détecter la première lectine en utilisant au moins le fait de détecter une fluorescence provenant du deuxième fluorophore, facultativement où le premier fluorophore est un type de fluorophore différent du deuxième fluorophore.

20. Le système de la revendication 19, où la première lectine comprend la concanavaline A (Con A), le septième sucre comprend la N-acétylgalactosamine (GalNAc), et la deuxième lectine comprend l'agglutinine de soja (SBA).

21. Le système de l'une quelconque des revendications 12 à 19, où :
la première lectine comprend la concanavaline A (Con A) et le premier sucre comprend le mannose (Man) ou le glucose (Glc) ;
la première lectine comprend l'agglutinine de germe de blé (WGA) et le premier sucre comprend la N-acétylglucosamine (GlcNAc) ou l'acide N-acétylneuraminique (NeuSAc) ;
la première lectine comprend l'agglutinine de soja (SBA) et le premier sucre comprend le galactose (Gal) ou la N-acétylgalactosamine (GalNAc) ;
la première lectine comprend la *Dolichos bifloris* (DBA) et le premier sucre comprend GalNAcα3GalNAc ou GalNAc ;
la première lectine comprend la ricine et le premier sucre comprend le galactose ;
la première lectine comprend l'agglutinine d'arachide (PNA) et le premier sucre comprend le galactose ou Galβ3GalNAcα (antigène T) ;
la première lectine comprend la *Pisum sativum* (PSA) et le premier sucre comprend le mannose ou le glucose ;
la première lectine comprend la *Lens culinaris* (LCA) et le premier sucre comprend le mannose ou le glucose ;
la première lectine comprend la *Galanthus nivalis* (GNA) et le premier sucre comprend le mannose ;
la première lectine comprend la *Solanum tuberosum* (STA) et le premier sucre comprend (GlcNAc)ₙ ;
la première lectine comprend le récepteur des asialoglycoprotéines (R-ASGP) H1 et le premier sucre comprend le galactose ;
la première lectine comprend la galectine-3 et le premier sucre comprend le galactose ;
la première lectine comprend la sialoadhésine et le premier sucre comprend Neu5Ac ;
la première lectine comprend le récepteur du mannose-6-phosphate dépendant des cations (CD-MPR) et le premier sucre comprend Man6P ; ou
la première lectine comprend la protéine C-réactive (CPR) et le premier sucre comprend le galactose, Gal6P, ou l'acide galacturonique.

22. Le système de l'une quelconque des revendications 12 à 21, où :
(i) le premier substrat comprend une bille ; et/ou
(ii) le premier sucre comprend un alkyl sucre ; et/ou
(iii) le premier sucre comprend un monosaccharide ; et/ou
(iv) le premier sucre comprend un disaccharide.
